(19) Europäisches Patentamt
European Patent Office
Office européen des brevets

(11) EP 1 570 860 A1

(12) EUROPEAN PATENT APPLICATION
published in accordance with Art. 158(3) EPC

(43) Date of publication:
07.09.2005 Bulletin 2005/36

(21) Application number: 03780739.3

(22) Date of filing: 12.12.2003

(51) Int Cl.[7]: A61K 45/00, A61K 31/49, A61K 31/503, A61K 31/5377, A61P 1/04, A61P 3/10, A61P 9/00, A61P 11/00, A61P 13/12, A61P 17/00, A61P 25/00, A61P 27/02, A61P 29/00, A61P 31/04, A61P 31/18, A61P 35/00, A61P 37/02, A61P 43/00

(86) International application number:
PCT/JP2003/015973

(87) International publication number:
WO 2004/054616 (01.07.2004 Gazette 2004/27)

(84) Designated Contracting States:
AT BE BG CH CY CZ DE DK EE ES FI FR GB GR HU IE IT LI LU MC NL PT RO SE SI SK TR
Designated Extension States:
AL LT LV MK

(30) Priority: 13.12.2002 JP 2002363013

(71) Applicant: ONO PHARMACEUTICAL CO., LTD.
Osaka 541-8526 (JP)

(72) Inventors:
- SHIBAYAMA, Shiro
Tsukuba-shi, Ibaraki 300-4247 (JP)
- SAGAWA, Kenji Ono Pharmaceutical Co., Ltd.
Mishima-gun, Osaka618 -8585 (JP)
- WATANABE, Noriki
Tsukuba-shi, Ibaraki 300-4247 (JP)
- TAKEDA, Kazuhiko
Tsukuba-shi, Ibaraki 300-4247 (JP)
- TADA, Hideaki
Tsukuba-shi, Ibaraki 300-4247 (JP)
- FUKUSHIMA, Daikichi
Tsukuba-shi, Ibaraki 300-4247 (JP)

(74) Representative: Henkel, Feiler & Hänzel
Möhlstrasse 37
81675 München (DE)

# (54) ANTAGONIST AND AGONIST BINDING TO STRONG BINDING SITE OF CHEMOKINE RECEPTOR

(57) An antagonist or an agonist which binds to a strong binding site of CCR5; a preventive and/or therapeutic agent for an allergic disease, an inflammatory disease, an immune disease and/or a cancer which comprises the same; a method for screening a compound which binds to a strong binding site of CCR5; a preventive and/or therapeutic agent for an allergic disease, an inflammatory disease, an immune disease and/or a cancer which comprises the antagonist or the agonist selected by the screening method; an antagonist or an agonist which binds to a strong binding site of a chemokine receptor; a preventive and/or therapeutic agent for an allergic disease, an inflammatory disease, an immune disease and/or a cancer which comprises the same; a method for screening a compound which binds to a strong binding site of a chemokine receptor; and a preventive and/or therapeutic agent for an allergic disease, an inflammatory disease, an immune disease and/or a cancer which comprises the antagonist or the agonist selected by the screening method. The antagonist or the agonist of the present invention is useful as a preventive and/or therapeutic agent for an allergic disease, an inflammatory disease, an immune disease and/or a cancer.

EP 1 570 860 A1

SEQUENCE LISTING

```
<110>  ONO PHARMACEUTICAL CO., LTD.

<120>  Antagonist and agonist which can be combined with strong binding site
of chemokine receptor.

<130>  ONF-4850PCT

<150>  JP 2002-363013
<151>  2002-12-13

<160>  2

<170>  PatentIn version 3.1

<210>  1
<211>  37
<212>  DNA
<213>  Artificial Sequence

<220>
<223>  hCCR5XbaI-F1(Forword primer)

<400>  1
agctagtcta gatccgttcc cctacaagaa actctcc                              37

<210>  2
<211>  37
<212>  DNA
<213>  Artificial Sequence

<220>
<223>  hCCR5XbaI-R1(Revese primer)

<400>  2
agctagtcta gagtgcacaa ctctgactgg gtcacca                              37
```

## Description

Technical Field

The present invention relates to (1) an antagonist or an agonist which binds to a strong binding site of CCR5; (2) a preventive and/or therapeutic agent for an allergic disease, an inflammatory disease, an immune disease and/or a cancer which comprises the same; (3) a method for screening a compound which binds to a strong binding site of CCR5; (4) a preventive and/or therapeutic agent for an allergic disease, an inflammatory disease, an immune disease and/or a cancer which comprises the antagonist or the agonist selected by the screening method, (5) an antagonist or an agonist which binds to a strong binding site of a chemokine receptor; (6) a preventive and/or therapeutic agent for an allergic disease, an inflammatory disease, an immune disease and/or a cancer which comprises the same; (7) a method for screening a compound which binds to a strong binding site of a chemokine receptor; and (8) a preventive and/or therapeutic agent for an allergic disease, an inflammatory disease, an immune disease and/or a cancer which comprises the antagonist or the agonist selected by the screening method.

Background Art

Chemokine is known as a basic protein having endogeneous leukocyte chemotactic and activating abilities and strong heparin-binding abilities. At present, it is considered that chemokine is related to not only the control of infiltration of specific leukocyte at the time of inflammations and immune responses but also the development and homing of lymphocyte under physiological conditions and migration of hemocyte precursor cells and somatic cells.
Differentiation, proliferation and cell death of hemocytes are controlled by various types of cytokine. In the living body, inflammations are found topically and differentiation, maturation and the like of lymphocytes are carried out at certain specified sites. That is, various necessary cells migrate into certain specified sites and accumulate therein to cause a series of inflammations and immune responses. Accordingly, migration of cells is also an indispensable phenomenon in addition to differentiation, proliferation and death of cells.
Migration of hemocytes in the living body starts firstly in the development stage by the shift of hematopoiesis started in the AGM region into permanent hematopoiesis in bone marrow via fetal liver. Furthermore, precursor cells of T cells and thymus dendritic cells migrate from the fetal liver into the bone marrow and then into the thymus gland and cytodifferentiate under thymus environment. The T cell which received clone selection migrates into secondary lymphoid tissues and takes part in an immune response in the periphery. The Langerhans' cell of the skin activated and differentiated by capturing an antigen migrates into the T cell region of a topical lymph node and activates naive T cell therein as a dendritic cell. The memory T cell performs its homing again into the lymph node via lymphatic and blood vessels. Also, B cell, T cell in the intestinal epithelium, γδ T cell, NKT cell and dendritic cell migrate from bone marrow without passing through the thymus gland and differentiate to take part in an immune response.
Chemokine is deeply related to the migration of these various cells. For example, MIP3β, SLC and its receptor CCR7 play an important role in the migration and homing of naive T cell, memory T cell and the mature dendritic cell which captured an antigen into a topical lymphoid tissue for the dendritic cells to encounter efficiently with the T cells. The T cell and dendritic cell necessary for controlling antigen-specific immune responses are hardly observed in the secondary lymph node of a PLT mouse having deficiency in the expression of SLC (*J. Exp. Med*., 189(3), 451 (1999)).
MDC, TARC and its receptor CCR4 play an important role in the migration of Th2 cell into topical sites in immune and inflammatory responses in which the Th2 cell is related. In a rat fluminant hepatitis model (P. acnes + LPS), an anti-TARC antibody suppressed increase of the amount of ALT in blood and increase of the expressing amounts of TNPα and FasL in the liver and also improved lethality of the rats (*J. Clin. Invest*., 102, 1933 (1998)). Also, an anti-MDC antibody decreased the number of eosinophils accumulated in the lung interstitium and suppressed airway hypersensitivity in a mouse OVA-induced airway hypersensitivity model (*J. Immunology*, 163, 403 (1999)).
MCP-1 and its receptor CCR2 are related to the infiltration of macrophage into inflammation sites. An anti-MCP-1 antibody showed an effect to suppress infiltration of monocyte and macrophage into glomerulus in a rat anti-Thy1.1 antibody glomerular nephritis model (*Kidney Int*., 51, 770 (1997)).
Thus, chemokine receptors are greatly related to the control of inflammation and immune responses through a mechanism in which they are expressed at certain specified periods in variously specific cells and the effector cells are accumulated in a region where chemokine is produced.
Acquired immunodeficiency syndrome (called AIDS) which is induced by human immunodeficiency virus (hereinafter referred to as "HIV") is one of the diseases of which their therapeutic methods are most earnestly desired in recent years. Once infection with HIV is completed in a CD4-positive cell which is a principal target cell, HIV repeats its proliferation in the body of the patient and, sooner or later, completely destroys T cell which takes charge of the immunological function. During this process, the immunological function is gradually reduced to cause fever, diarrhea, lymph node enlargement and the like various immunodeficiency conditions which are apt to cause complications with

pneumocystis carinii pneumonia and the like various opportunistic infections. Such conditions are the onset of AIDS, and it is well known that they induce and worsen Kaposi sarcoma and the like malignant tumors.

As the recent preventive and therapeutic methods for AIDS, attempts have been made to, e.g., (1) inhibit growth of HIV by the administration of a reverse transcriptase inhibitor or a protease inhibitor and (2) prevent or alleviate opportunistic infections by the administration of a drug having immunopotentiation activity.

Helper T cells which take charge of the central of immune system are mainly infected with HIV. It is known since 1985 that HIV uses the membrane protein CD4 expressing on the membrane of T cells in the infection (Cell, 52, 631 (1985)). The CD4 molecule is composed of 433 amino acid residues, and its expression can be found in macrophages, some B cells, vascular endothelial cells, Langerhans' cells in skin tissues, dendritic cells in lymphoid tissues, glia cells of the central nervous system and the like, in addition to the mature helper T cells. However, since it has been revealed that the infection with HIV is not completed by the CD4 molecule alone, a possibility has been suggested on the presence of factors other than the CD4 molecule, which are related to the infection of cells with HIV.

In 1996, a cell membrane protein called Fusin was identified as a factor other than the CD4 molecule, which is related to the HIV infection (*Science*, 272, 872 (1996)). It was confirmed that this Fusin molecule is a receptor (namely, CXCR4) of stromal derived factor-1 (hereinafter referred to as "SDF-1"). In addition, it was confirmed also in vitro that the SDF-1 specifically inhibits infection of T cell tropic (X4) HIV (*Nature*, 382, 829 (1996), *Nature*, 382, 833 (1996)). That is, it is considered that the HIV infection was inhibited by the binding of SDF-1 to CXCR4 preceding HIV, thereby depriving HIV of a foothold for infecting cells.

Also at that time, it was discovered that another chemokine receptor CCR5, which is a receptor of RANTES, MIP-1$\alpha$ and MIP-1$\beta$, is also used at the time of the infection with a macrophage tropic (R5) HIV (*Science*, 272, 1955 (1996)).

Accordingly, substances which can compete with CXCR4 and CCR5 for HIV, or which can bind to HIV virus thus causing the virus unable to bind to CXCR4 and CCR5, could become HIV infection inhibitors. Also, there is a case in which a low molecular compound initially discovered as an HIV infection inhibitor was actually a CXCR4 antagonist (*Nature Medicine*, 4, 72 (1998)).

Based on the above, it is considered that the chemokine/chemokine receptors are deeply related to the inflammation, immune disease or HIV infection. For example, it is considered that they are related to various inflammatory diseases, asthma, atopic dermatitis, nettle rash, allergic diseases (allergic bronchopulmonary aspergillosis, allergic eosinophilic gastroenteritis and the like), nephritis, nephropathy, hepatitis, arthritis, chronic rheumatoid arthritis, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune diseases, transplant rejection, cancer metastasis, acquired immunodeficiency syndrome and the like.

In the specification of WO01/40227, it is disclosed that triazaspiro[5.5]undecane derivatives of the formula (A), quaternary ammonium salts thereof, N-oxides thereof or non-toxic salts thereof which regulate the effect of chemokine/chemokine receptor.

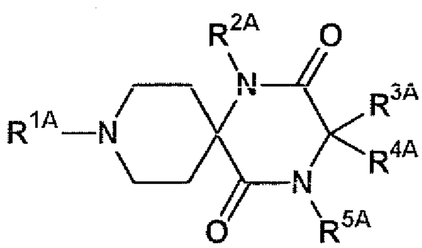

(A)

[wherein $R^{1A}$ is,

(1) hydrogen,
(2) C1-18 alkyl,
(3) C2-18 alkenyl,
(4) C2-18 alkynyl,
(5) $-COR^{6A}$,
(6) $-CONR^{7A}R^{8A}$,
(7) $-COOR^{9A}$,
(8) $-SO_2R^{10A}$,
(9) $-COCOOR^{11A}$,
(10) $-CONR^{12A}COR^{13A}$,

(11) Cyc $1^A$, or
(12) C1-18 alkyl, C2-18 alkenyl or C2-18 alkynyl substituted by 1-5 of optionally selected from (a) halogen, (b) $-CONR^{7A}R^{8A}$, (c) $-COOR^{9A}$, (d) $-OR^{14A}$, (e) - $SR^{15A}$, (f) $-NR^{16A}R^{17A}$, (g) $-NR^{18A}COR^{19A}$, (h) $-SO_2NR^{20A}R^{21A}$, (i) $-OCOR^{22A}$, (j) - $NR^{23A}SO_2R^{24A}$, (k) $-NR^{25A}COOR^{26A}$, (l) $-NR^{27A}CONR^{28A}R^{29A}$, (m) Cyc $1^A$, (n) keto and (o) $-N(SO_2R^{24A})_2$,

(wherein $R^{6A}$-$R^{9A}$, $R^{11A}$-$R^{21A}$, $R^{23A}$, $R^{25A}$ and $R^{27A}$-$R^{29A}$ are, each independently,

(1) hydrogen,
(2) C1-8 alkyl,
(3) C2-8 alkenyl,
(4) C2-8 alkynyl,
(5) Cyc $1^A$, or
(6) C1-8 alkyl, C2-8 alkenyl or C2-8 alkynyl substituted by 1-5 of optionally selected from (a) Cyc $1^A$, (b) halogen, (c) $-OR^{30A}$, (d) $-SR^{31A}$, (e) $-NR^{32A}R^{33A}$, (f) $-COOR^{34A}$, (g) $-CONR^{35A}R^{36A}$, (h) $-NR^{37A}COR^{38A}$, (i) $-NR^{39A}SO_2R^{40A}$ and (j) - $N(SO_2R^{40A})_2$, or

$R^{7A}$ and $R^{8A}$, $R^{20A}$ and $R^{21A}$, $R^{28A}$ and $R^{29A}$, taken together, are (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{195A}$-(C2-6 alkylene)-, (wherein $R^{195A}$ is hydrogen, C1-8 alkyl, pherlyl or C1-8alkyl substituted by phenyl.),
$R^{10A}$, $R^{22A}$, $R^{24A}$ and $R^{26A}$ are, each independently,

(1) C1-8 alkyl,
(2) C2-8 alkenyl,
(3) C2-8 alkynyl,
(4) Cyc $1^A$, or
(5) C1-8 alkyl, C2-8 alkenyl or C2-8 alkynyl substituted by 1-5 of optionally selected from (a) Cyc $1^A$, (b) halogen, (c) $-OR^{30A}$, (d) $-SR^{31A}$, (e) $-NR^{32A}R^{33A}$, (f) $-COOR^{34A}$, (g) $-CONR^{35A}R^{36A}$, (h) $-NR^{37A}COR^{38A}$, (i) $-NR^{39A}SO_2R^{40A}$ and (j) - $N(SO_2R^{40A})_2$,

(wherein $R^{30A}$-$R^{37A}$ and $R^{39A}$ are, each independently, hydrogen, C1-8 alkyl, Cyc $1^A$ or C1-8 alkyl substituted by Cyc $1^A$, or
$R^{35A}$ and $R^{36A}$, taken together, are (1) C2-6 alkylene, (2) -(C2-6 alkylene)-O-(C2-6 alkylene)-, (3) -(C2-6 alkylene)-S-(C2-C alkylene)- or (4) -(C2-6 alkylene)-$NR^{196A}$-(C2-6 alkylene)-, (wherein $R^{196A}$ is hydrogen, C1-8 alkyl, phenyl or C1-8 alkyl substituted by phenyl.),
$R^{38A}$ and $R^{40A}$ are, each independently, C1-8 alkyl, Cyc $1^A$ or C1-8 alkyl substituted by Cyc $1^A$.)
Cyc $1^A$ is C3-15 mono-, bi- or tri-(fused or spiro)carbocyclic ring or 3-15 membered mono-, bi- or tri-(fused or spiro)cyclic hetero ring containing 1-4 nitrogen atom(s), 1-3 oxygen atom(s) and/or 1-3 sulfur atom(s).

With the proviso that, Cyc $1^A$ may be optionally substituted by 1-5 of $R^{51A}$, $R^{51A}$ is,

(1) C1-8 alkyl,
(2) C2-8 alkenyl,
(3) C2-8 alkynyl,
(4) halogen,
(5) nitro,
(6) trifluoromethyl,
(7) trifluoromethoxy,
(8) nitrile,
(9) keto,
(10) Cyc $2^A$
(11) $-OR^{52A}$,
(12) $-SR^{53A}$,
(13) $-NR^{54A}R^{55A}$,
(14) $-COOR^{56A}$,
(15) $-CONR^{57A}R^{58A}$,
(16) $-NR^{59A}COR^{60A}$,
(17) $-SO_2NR^{61A}R^{62A}$,

(18) $-OCOR^{63A}$,
(19) $-NR^{64A}SO_2R^{65A}$,
(20) $-NR^{66A}COOR^{67A}$,
(21) $-NR^{68A}CONR^{69A}R^{70A}$,
(22) $-B(OR^{71A})_2$,
(23) $-SO_2R^{72A}$,
(24) $-N(SO_2R^{72A})_2$, or
(25) C1-8 alkyl, C2-8 alkenyl or C2-8 alkynyl substituted by 1-5 of optionally selected from (a) halogen, (b) Cyc $2^A$, (c) $-OR^{52A}$, (d) $-SR^{53A}$, (e) $-NR^{54A}R^{55A}$, (f) $-COOR^{56A}$, (g) $-CONR^{57A}R^{58A}$, (h) $-NR^{59A}COR^{60A}$, (i) $-SO_2NR^{61A}R^{62A}$, (j) $-OCOR^{63A}$, (k) $-NR^{64A}SO_2R^{65A}$, (l) $-NR^{66A}COOR^{67A}$, (m) $-NR^{68A}CONR^{69A}R^{70A}$, (n) $-B(OR^{71A})_2$, (o) $-SO_2R^{72A}$ and (p) $-N(SO_2R^{72A})_2$.)
(wherein $R^{52A}$-$R^{62A}$, $R^{64A}$,$R^{66A}$ and $R^{68A}$-$R^{71A}$ are, each independently, (1) hydrogen, (2) C1-8 alkyl, (3) C2-8 alkenyl, (4) C2-8 alkynyl, 5) Cyc $2^A$ or (6) C1-8 alkyl, C2-8 alkenyl or C2-8 alkynyl substituted by Cyc $2^A$, $-OR^{73A}$, $-COOR^{74A}$, $-NR^{75A}R^{76A}$, or

$R^{57A}$ and $R^{58A}$, $R^{61A}$ and $R^{62A}$, $R^{69A}$ and $R^{70A}$, taken together, are (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{197A}$-(C2-6 alkylene)-, (wherein $R^{197A}$ is hydrogen, C1-8 alkyl, phenyl or C1-8alkyl substituted by phenyl.),
$R^{63A}$, $R^{65A}$, $R^{67A}$ and $R^{72A}$ are, each independently, (1) C1-8 alkyl, (2) C2-8 alkenyl, (3) C2-8 alkynyl, (4) Cyc $2^A$ or (5) C1-8 alkyl, C2-8 alkenyl or C2-8 alkynyl substituted by Cyc $2^A$, $-OR^{73A}$, $-COOR^{74A}$ or $-NR^{75A}R^{76A}$, (wherein $R^{73A}$-$R^{76A}$ are independently hydrogen, C1-8 alkyl, Cyc $2^A$ or C1-8 alkyl substituted by Cyc $2^A$.)
Cyc $2^A$ has the same meaning as Cyc 1. With the proviso that, Cyc $2^A$ may be optionally substituted by 1-5 of $R^{77A}$, $R^{77A}$ is,

(1) C1-8 alkyl,
(2) halogen,
(3) nitro,
(4) trifluoromethyl,
(5) trifluoromethoxy,
(6) nitrile,
(7) $-OR^{78A}$,
(8) $-NR^{79A}R^{80A}$,
(9) $-COOR^{81A}$,
(10) $-SR^{82A}$,
(11) $-CONR^{83A}R^{84A}$,
(12) C2-8 alkenyl,
(13) C2-8 alkynyl,
(14) keto,
(15) Cyc $6^A$,
(16) $-NR^{161A}COR^{162A}$,
(17) $-SO_2NR^{163A}R^{164A}$,
(18) $-OCOR^{165A}$,
(19) $-NR^{166A}SO_2R^{167A}$,
(20) $-NR^{168A}COOR^{169A}$,
(21) $-NR^{170A}CONR^{171A}R^{172A}$,
(22) $-SO_2R^{173A}$,
(23) $-N(SO_2R^{167A})_2$, or
(24) C1-8 alkyl, C2-8 alkenyl or C2-8 alkynyl substituted by 1-5 of optionally selected from (a) halogen, (b) $-OR^{78A}$, (c) $-NR^{79A}R^{80A}$, (d) $-COOR^{81A}$, (e) $-SR^{82A}$, (f) $-CONR^{83A}R^{84A}$, (g) keto, (h) Cyc $6^A$, (i) $-NR^{161A}COR^{162A}$, (j) $-SO_2NR^{163A}R^{164A}$, (k) $-OCOR^{165A}$, (1) $-NR^{166A}SO_2R^{167A}$, (m) $-NR^{168A}COOR^{169A}$, (n) $-NR^{170A}CONR^{171A}R^{172A}$, (o)$-SO_2R^{173A}$ or (p) $-N(SO_2R^{167A})_2$.)

(wherein $R^{78A}$-$R^{84A}$, $R^{161A}$-$R^{164A}$, $R^{166A}$, $R^{168A}$ and $R^{170A}$-$R^{172A}$ are, each independently, (a.) hydrogen, (b) C1-8 alkyl, (c) C2-8 alkenyl, (d) C2-8 alkynyl, (e) Cyc $6^A$ or (f) C1-8 alkyl, C2-8 alkenyl or C2-8 alkynyl substituted by Cyc $6^A$, $-OR^{174A}$, $-COOR^{175A}$, $-NR^{176A}R^{177A}$ or $-CONR^{178A}R^{179A}$, or

$R^{83A}$ and $R^{84A}$, $R^{163A}$ and $R^{164A}$, $R^{171A}$ and $R^{172A}$, taken together, are (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{198A}$-(C2-6 alkylene)-,

(wherein $R^{198A}$ is hydrogen, C1-8 alkyl, phenyl or C1-8 alkyl substituted by phenyl.),
$R^{165A}$, $R^{167A}$, $R^{169A}$ and $R^{173A}$ are, each independently, (a) C1-8 alkyl, (b) C2-8 alkenyl, (c) C2-8 alkynyl, (d) Cyc $6^A$ or (e) C1-8 alkyl, C2-8 alkenyl, C2-8 alkynyl substituted by Cyc $6^A$, $-OR^{174A}$, $-COOR^{175A}$, $-NR^{176A}R^{177A}$ or $-CONR^{178A}R^{179A}$.)
(wherein $R^{174A}$-$R^{177A}$ are, each independently, (1) hydrogen, (2) C1-8 alkyl, (3) Cyc $6^A$ or (4) C1-8 alkyl substituted by Cyc $6^A$, or
$R^{178A}$ and $R^{179A}$, taken together, are (1) C2-6 alkylene, (2) -(C2-6 alkylene)-O-(C2-6 alkylene)-, (3) -(C2-6 alkylene) -S-(C2-6 alkylene)- or (4) -(C2-6 alkylene)-$NR^{199A}$-(C2-6 alkylene)- (wherein $R^{199A}$ is hydrogen, C1-8 alkyl, phenyl or C1-8 alkyl substituted by phenyl.),
Cyc $6^A$ is C3-8 mono-carbocyclic ring or 3-8 membered mono-cyclic hetero ring containing 1-4 nitrogen atom(s), 1-2 oxygen atom(s) and/or 1-2 sulfur atom(s).

With the proviso that, Cyc $6^A$ may be optionally substituted by 1-5 of $R^{180A}$, $R^{180A}$ is,

(1) C1-8 alkyl,
(2) halogen,
(3) nitro,
(4) trifluoromethyl,
(5) trifluoromethoxy,
(6) nitrile,
(7) $-OR^{181A}$,
(8) $-NR^{182A}R^{183A}$,
(9) $-COOR^{184A}$,
(10) $-SR^{185A}$, or
(11) $-CONR^{186A}R^{187A}$,

(wherein $R^{181A}$-$R^{187A}$ are, each independently, (1) hydrogen, (2) C1-8 alkyl, (3) phenyl or (4) C1-8 alkyl substituted by phenyl,

$R^{182A}$ and $R^{183A}$, $R^{186A}$ and $R^{187A}$, taken together, are (1) C2-6 alkylene, (2) - (C2-6 alkylene)-O-(C2-6 alkylene)-, (3) -(C2-6 alkylene)-S-(C2-6 alkylene)- or (4)-(C2-6 alkylene)-$NR^{200A}$-(C2-6 alkylene)-, (wherein $R^{200A}$ is hydrogen, C1-8 alkyl, phenyl or C1-8 alkyl substituted by phenyl.).),
$R^{2A}$ is,

(1) hydrogen,
(2) C1-8 alkyl,
(3) C2-8 alkenyl,
(4) C2-8 alkynyl,
(5) $-OR^{90A}$,
(6) Cyc $3^A$, or
(7) C1-8 alkyl, C2-8 alkenyl or C2-8 alkynyl substituted by 1-5 of optionally selected from (a) halogen, (b) $-OR^{90A}$, (c) $-SR^{91A}$, (d) $-NR^{92A}R^{93A}$, (e) - $COOR^{94A}$, (f) $-CONR^{95A}R^{96A}$, (g) $-NR^{97A}COR^{98A}$, (h) $-SO_2NR^{99A}R^{100A}$, (i) $-OCOR^{101A}$, (j) $-NR^{102A}SO_2R^{103A}$, (k) $-NR^{104A}COOR^{105A}$, (l) $-NR^{106A}CONR^{107A}R^{108A}$, (m) Cyc $3^A$, (n) keto and (o) $-N(SO_2R^{103A})_2$,

(wherein $R^{90A}$-$R^{100A}$, $R^{102A}$,$R^{104A}$ and $R^{106A}$-$R^{108A}$ are, each independently, (1) hydrogen, (2) C1-8 alkyl, (3) C2-8 alkenyl, (4) C2-8 alkynyl, (5) Cyc $3^A$ or (6) C1-8 alkyl, C2-8 alkenyl or C2-8 alkynyl substituted by Cyc $3^A$, or
$R^{95A}$ and $R^{96A}$, $R^{99A}$ and $R^{100A}$, $R^{107A}$ and $R^{108A}$, taken together, are (1) C2-6 alkylene, (2) -(C2-6 alkylene) -O-(C2-6 alkylene)-, (3) -(C2-6 alkylene)-S-(C2-6 alkylene)- or (4) -(C2-6 alkylene)-$NR^{201A}$-(C2-6 alkylene)-, (wherein $R^{201A}$ is hydrogen, C1-8 alkyl, phenyl or C1-8 alkyl substituted by phenyl.),
$R^{101A}$, $R^{103A}$ and $R^{105A}$ are, each independently, (1) C1-8 alkyl, (2) C2-8 alkenyl, (3) C2-8 alkynyl or (4) Cyc $3^A$, or C1-8 alkyl, C2-8 alkenyl or C2-8 alkynyl substituted by Cyc $3^A$,
Cyc $3^A$ has the same meaning as Cyc $1^A$.
With the proviso that, Cyc $3^A$ may be optionally substituted by 1-5 of $R^{109A}$,
$R^{109A}$ has the same meaning as $R^{51A}$.)
$R^{3A}$ and $R^{4A}$ are, each independently,

(1) hydrogen,

(2) C1-8 alkyl,
(3) C2-8 alkenyl,
(4) C2-8 alkynyl,
(5) $-COOR^{120A}$,
(6) $-CONR^{121A}R^{122A}$,
(7) Cyc $4^{A}$, or
(8) C1-8 alkyl, C2-8 alkenyl or C2-8 alkynyl substituted by 1-5 of optionally selected from (a) halogen, (b) nitrile, (c) Cyc $4^{A}$, (d) $-COOR^{120A}$, (e) - $CONR^{121A}R^{122A}$, (f) $-OR^{123A}$, (g) $-SR^{124A}$, (h) $-NR^{125A}R^{126A}$, (i) $-NR^{127A}COR^{128A}$, (j) - $SO_2NR^{129A}R^{130A}$, (k) $-OCOR^{131A}$, (l) $-NR^{132A}SO_2R^{133A}$, (m) $-NR^{134A}COOR^{135A}$, (n) - $NR^{136A}CONR^{137A}R^{138A}$, (o) $-S-SR^{139A}$, (p) $-NHC(=NH)NHR^{140A}$, (q) keto, (r) - $NR^{145A}CONR^{146A}COR^{147A}$ and (s) $-N(SO_2R^{133A})_2$,

(wherein $R^{120A}$-$R^{130A}$, $R^{132A}$, $R^{134A}$, $R^{136A}$-$R^{138A}$, $R^{145A}$ and $R^{146A}$ are, each independently, (1) hydrogen, (2) C1-8 alkyl, (3) C2-8 alkenyl, (4) C2-8 alkynyl, (5) Cyc $4^{A}$ or (6) C1-8 alkyl, C2-8 alkenyl or C2-8 alkynyl substituted by Cyc $4^{A}$, halogen, - $OR^{148A}$, $-SR^{149A}$, $-COOR^{150A}$ or $-NHCOR^{141A}$, or
$R^{121A}$ and $R^{122A}$, $R^{129A}$ and $R^{130A}$, $R^{137A}$ and $R^{138A}$, taken together, are (1) C2-6 alkylene, (2) -(C2-6 alkylene) -O-(C2-6 alkylene)-, (3) -(C2-6 alkylene)-S-(C2-6 alkylene)- or (4) -(C2-6 alkylene)-$NR^{202A}$-(C2-6 alkylene)-, (wherein $R^{202A}$ is hydrogen, C1-8 alkyl, phenyl or C1-8 alkyl substituted by phenyl.),
$R^{131A}$, $R^{133A}$, $R^{135A}$, $R^{139A}$ and $R^{147A}$ are, each independently, (1) C1-8 alkyl, (2) C2-8 alkenyl, (3) C2-8 alkynyl, (4) Cyc $4^{A}$ or (5) C1-8 alkyl, C2-8 alkenyl or C2-8 alkynyl substituted by Cyc $4^{A}$, halogen, $-OR^{148A}$, $-SR^{149A}$, $-COOR^{150A}$ or $-NHCOR^{141A}$, $R^{140A}$ is hydrogen, $-COOR^{142A}$ or $-SO_2R^{143A}$,
(wherein $R^{141A}$-$R^{143A}$ are, each independently, (1) C1-8 alkyl, (2) C2-8 alkenyl, (3) C2-8 alkynyl, (4) Cyc $4^{A}$ or (5) C1-8 alkyl, C2-8 alkenyl or C2-8 alkynyl substituted by Cyc $4^{A}$,
$R^{148A}$-$R^{150A}$ are, each independently, (1) hydrogen, (2) C1-8 alkyl, (3) C2-8 alkenyl, (4) C2-8 alkynyl, (5) Cyc $4^{A}$ or (6) C1-8 alkyl, C2-8 alkenyl or C2-8 alkynyl substituted by Cyc $4^{A}$,
Cyc $4^{A}$ has the same meaning as Cyc $1^{A}$.
With the proviso that, Cyc $4^{A}$ may be optionally substituted by 1-5 of $R^{144A}$,
$R^{144A}$ has the same meaning as $R^{51A}$.) or
$R^{3A}$ and $R^{4A}$, taken together, are

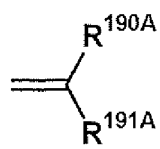

(wherein $R^{190A}$ and $R^{191A}$ are, each independently, the same meaning as $R^{3A}$ or $R^{4A}$.),
$R^{5A}$ is,

(1) hydrogen,
(2) C1-8 alkyl,
(3) Cyc $5^{A}$, or
(4) C1-8alkyl substituted by Cyc $5^{A}$.

(wherein Cyc $5^{A}$ has the same meaning as Cyc $1^{A}$.
With the proviso that, Cyc $5^{A}$ is optionally substituted by 1-5 of $R^{160A}$,
$R^{160A}$ has the same meaning as $R^{51A}$.)]

And also, in the specification of WO02/74770, it is disclosed that triazaspiro [5.5] undecane derivatives of the formula (B), quaternary ammonium salts thereof, N-oxides thereof or non-toxic salts thereof which regulate the effect of chemokine/chemokine receptor.

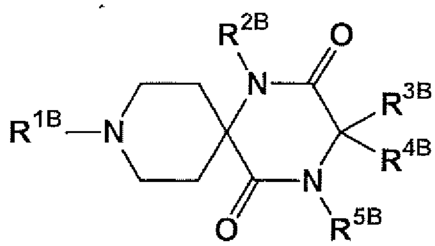

**(B)**

[wherein $R^{1B}$ is (1) or (2) :

(1) 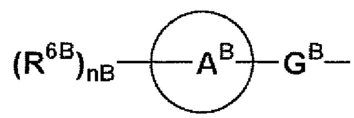 ,

(2) 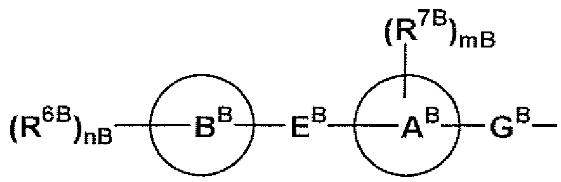

(wherein $G^B$ is, a single bond, C1-4 alkylene, C2-4 alkenylene, or -CO-,

$A^B$ ring is (1) C5-10 membered mono- or bi-carbocyclic ring or (2) 5-10 membered mono- or bi-cyclic hetero ring containing 1-2 nitrogen atom(s) and/or 1-2 oxygen atom(s),
$R^{6B}$ is,

(1) C1-4 alkyl,
(2) halogen,
(3) nitrile,
(4) trifluoromethyl,
(5) $-OR^{8B}$,
(6) $-SR^{9B}$,
(7) $-NR^{10B}R^{11B}$,
(8) $-COOR^{12B}$,
(9) $-CONR^{13B}R^{14B}$,
(10) $-SO_2NR^{15B}R^{16B}$,
(11) $-NR^{17B}SO_2R^{18B}$,
(12) $-S(O)R^{19B}$,
(13) $-SO_2R^{20B}$,
(14) $-N(SO_2R^{21B})_2$,
(15) C1-4 alkyl substituted by one of optionally selected from (a) $-OR^{8B}$, (b) $-NR^{10B}R^{11B}$, and (c) Cyc $1^B$, or
(16) $-NR^{27B}COR^{28B}$,

wherein $R^{8B}$-$R^{17B}$ are, each independently mono substituted C1-4 alkyl selected from (1) hydrogen, (2) C1-4 alkyl, (3) Cyc $1^B$, (4) $-OR^{22B}$, and (5) mono substituted C1-4 alkyl selected from (a) $-OR^{22B}$, (b) $-NR^{23B}R^{24B}$, (c) $-COOR^{25B}$, and (d) Cyc $1^B$, or
$R^{10B}$ and $R^{11B}$, $R^{13B}$ and $R^{14B}$, $R^{15B}$ and $R^{16B}$, taken together, are 5-6 membered mono-cyclic hetero ring containing 1-2 nitrogen atom(s) and/or an oxygen atom (With the proviso that, 5-6 membered mono-cyclic hetero ring may be optionally substituted by C1-4 alkyl or hydroxy.),
$R^{22B}$-$R^{25B}$ are, each independently, (1) hydrogen, (2) C1-4 alkyl or (3) C1-4 alkyl substituted by C1-4 alkoxy,
$R^{23B}$ and $R^{24B}$ , taken together, is 5-6 membered mono-cyclic hetero ring containing 1-2 nitrogen atom(s) and/or an oxygen atom (With the proviso that, 5-6 membered mono-cyclic hetero ring may be optionally substituted by

C1-4 alkyl or hydroxy.),
$R^{18B}$-$R^{21B}$ are, each independently, C1-4 alkyl,
$R^{27B}$ is (1) hydrogen, (2) C1-4 alkyl, (3) Cyc $1^B$ or (4) C1-4 alkyl substituted by one of optionally selected from (a) -$OR^{22B}$ , (b) -$NR^{23B}R^{24B}$, (c) -$COOR^{25B}$, and (d) Cyc 1B,
$R^{28B}$ is (1) C1-4 alkyl, (2) Cyc $1^B$ or (3) C1-4 alkyl substituted by one of optionally selected from (a) -$OR^{22B}$ , (b) -$NR^{23B}R^{24B}$, (c) -$COOR^{25B}$, and (d) Cyc $1^B$,
Cyc $1^B$ is (1) C5-6 membered mono-carbocyclic ring or (2) 5-6 membered mono-cyclic hetero ring containing 1-2 nitrogen atom(s) and/or an oxygen atom (With the proviso that, C5-6 membered mono-carbocyclic ring or 5-6 membered mono-cyclic hetero ring may be optionally substituted by C1-4 alkoxy, halogen, or -$COOR^{29\,B}$ (wherein $R^{29\,B}$ is (1) hydrogen, (2) C1-4 alkyl, (3) Cyc $1^B$, or (4) C1-4 alkyl substituted by one of optionally selected from (a) -$OR^{22B}$, (b) -$NR^{23B}R^{24B}$, (c) -$COOR^{25B}$, and (d) Cyc 1B.).),
$E^B$ is, a single bond, -O-, -S-, -CO-, or -CHOH-,
$B^B$ ring is (1) C5-6 membered mono-carbocyclic ring or (2) 5-6 membered mono- cyclic hetero ring containing 1-2 nitrogen atom(s) and/or an oxygen atom,
$R^{7B}$ is C1-4 alkyl or halogen,
nB is a number from 0 or 1 to 4 and mB is a number from 0 or 1 to 4.),
$R^{2B}$ is (1) C1-4 alkyl, (2) C2-4 alkynyl, or (3) C1-4 alkyl substituted by one of optionally selected from (a) -$OR^{30B}$, (b) -$NR^{31B}R^{32B}$ and (c) Cyc $3^B$ (wherein $R^{30B}$-$R^{32B}$ are, each independently, (1) hydrogen, (2) C1-4 alkyl, (3) Cyc $3^B$ or (4) C1-4 alkyl substituted by Cyc $3^B$ wherein Cyc $3^B$ is (1) C5-6 membered mono-carbocyclic ring or (2) 5-6 membered mono-cyclic hetero ring containing 1-2 nitrogen atom(s) and/or an oxygen atom (With the proviso that, C5-6 membered mono-carbocyclic ring or 5-6 membered mono-cyclic hetero ring may be optionally substituted by C1-4 alkoxy.).),
$R^{3B}$ and $R^{4B}$ are, each independently, (1) hydrogen, (2) C1-4 alkyl, or (3) C1-4 alkyl substituted by 1-2 of optionally selected from (a) Cyc $2^B$, and (b) hydroxy (wherein Cyc $2^B$ is (1) C5-6 membered mono-carbocyclic ring or (2) 5-6 membered mono-cyclic hetero ring containing 1-2 nitrogen atom(s) and/or an oxygen atom.), or taken together, is

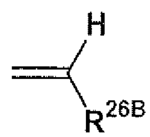

(wherein $R^{26B}$ is C1-4 alkyl or Cyc $2^B$),
and $R^{5B}$ is hydrogen or C1-4 alkyl.]

Moreover, in the specification of WO02/74769, it is disclosed that the compounds of the formula (C), quaternary ammonium salts thereof, N-oxides thereof or non-toxic salts thereof, as the agent for prevention and/or treatment for human immnodeficiency virus (hereinafter referred to as "HIV") infection or acquired immune deficiency syndrome (called AIDS) induced by the infection.

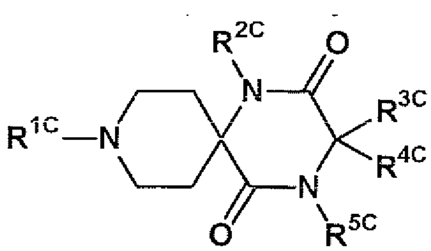

(C)

[wherein $R^{1C}$ is,

(1) hydrogen,
(2) C1-18 alkyl,
(3) C2-18 alkenyl,
(4) C2-18 alkynyl,
(5) -$COR^{6C}$,
(6) -$CONR^{7C}R^{8C}$,
(7) -$COOR^{9C}$,

(8) $-SO_2R^{10C}$,
(9) $-COCOOR^{11C}$,
(10) $-CONR^{12C}COR^{13C}$,
(11) Cyc $1^C$, or
(12) C1-18 alkyl, C2-18 alkenyl or C2-18 alkynyl substituted by 1-5 of optionally selected from (a) halogen, (b) $-CONR^{7C}R^{8C}$, (c) $-COOR^{9C}$, (d) $-OR^{14C}$, (e) $-SR^{15C}$, (f) $-NR^{16C}R^{17C}$, (g) $-NR^{18C}COR^{19C}$, (h) $-SO_2NR^{20C}R^{21C}$, (i) $-OCOR^{22C}$, (j) $-NR^{23C}SO_2R^{24C}$, (k) $-NR^{25C}COOR^{26C}$, (l) $-NR^{27C}CONR^{28C}R^{29C}$, (m) Cyc $1^C$, (n) keto and (o) $-N(SO_2R^{24C})_2$,
(wherein $R^{6C}$-$R^{9C}$, $R^{11C}$-$R^{21C}$, $R^{23C}$, $R^{25C}$ and $R^{27C}$-$R^{29C}$ are, each independently,

(1) hydrogen,
(2) C1-8 alkyl,
(3) C2-8 alkenyl,
(4) C2-8 alkynyl,
(5) Cyc $1^C$, or
(6) C1-8 alkyl, C2-8 alkenyl or C2-8 alkynyl substituted by 1-5 of optionally selected from (a) Cyc $1^C$, (b) halogen, (c) $-OR^{30C}$, (d) $-SR^{31C}$, (e) $-NR^{32C}R^{33C}$, (f) $-COOR^{34C}$, (g) $-CONR^{35C}R^{36C}$, (h) $-NR^{37C}COR^{38C}$, (i) $-NR^{39C}SO_2R^{40C}$ and (j) $-N(SO_2R^{40C})_2$, or

$R^{7C}$ and $R^{8C}$, $R^{20C}$ and $R^{21C}$, $R^{28C}$ and $R^{29C}$, taken together, are (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{195C}$-(C2-6 alkylene)-, (wherein $R^{195C}$ is hydrogen, C1-8 alkyl, phenyl or C1-8alkyl substituted by phenyl.),
$R^{10C}$, $R^{22C}$, $R^{24C}$ and $R^{26C}$ are, each independently,

(1) C1-8 alkyl,
(2) C2-8 alkenyl,
(3) C2-8 alkynyl,
(4) Cyc $1^C$, or
(5) C1-8 alkyl, C2-8 alkenyl or C2-8 alkynyl substituted by 1-5 of optionally selected from (a) Cyc $1^C$, (b) halogen, (c) $-OR^{30C}$, (d) $-SR^{31C}$, (e) $-NR^{32C}R^{33C}$, (f) $-COOR^{34C}$, (g) $-CONR^{35C}R^{36C}$, (h) $-NR^{37C}COR^{38C}$, (i) $-NR^{39C}SO_2R^{40C}$ and (j) $-N(SO_2R^{40C})_2$,
(wherein $R^{30c}$-$R^{37C}$ and $R^{39C}$ are, each independently, hydrogen, C1-8 alkyl, Cyc $1^C$ or C1-8 alkyl substituted by Cyc $1^C$, or

$R^{35C}$ and $R^{36C}$, taken together, are (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{196C}$-(C2-6 alkylene)-, (wherein $R^{196C}$ is hydrogen, C1-8 alkyl, phenyl or C1-8 alkyl substituted by phenyl.),
$R^{38C}$ and $R^{40C}$ are, each independently C1-8 alkyl, Cyc $1^C$ or C1-8 alkyl substituted by Cyc $1^C$.)
Cyc $1^C$ is C3-15 mono-, bi- or tri-(fused or spiro)carbocyclic ring or 3-15 membered mono-, bi- or tri-(fused or spiro) cyclic hetero ring containing 1-4 nitrogen atom(s), 1-3 oxygen atom(s) and/or 1-3 sulfur atom(s). With the proviso that, Cyc $1^C$ may be optionally substituted by 1-5 of $R^{51C}$,
$R^{51C}$ is,

(1) C1-8 alkyl,
(2) C2-8 alkenyl,
(3) C2-8 alkynyl,
(4) halogen,
(5) nitro,
(6) trifluoromethyl,
(7) trifluoromethoxy,
(8) nitrile,
(9) keto,
(10) Cyc $2^C$
(11) $-OR^{52C}$,
(12) $-SR^{53C}$,
(13) $-NR^{54C}R^{55C}$,
(14) $-COOR^{56C}$,
(15) $-CONR^{57C}R^{58C}$,

(16) $-NR^{59C}COR^{60C}$,
(17) $-SO_2NR^{61C}R^{62C}$,
(18) $-OCOR^{63C}$,
(19) $-NR^{64C}SO_2R^{65C}$,
(20) $-NR^{66C}COOR^{67C}$,
(21) $-NR^{68C}CONR^{69C}R^{70C}$,
(22) $-B(OR^{71C})_2$,
(23) $-SO_2R^{72C}$,
(24) $-N(SO_2R^{72C})_2$, or
(25) C1-8 alkyl, C2-8 alkenyl or C2-8 alkynyl substituted by 1-5 of optionally selected from (a) halogen, (b) Cyc $2^C$, (c) $-OR^{52C}$, (d) $-SR^{53C}$, (e) $-NR^{54C}R^{55C}$, (f) $-COOR^{56C}$, (g) $-CONR^{57C}R^{58C}$, (h) $-NR^{59C}COR^{60C}$, (i) $-SO_2NR^{61C}R^{62C}$, (j) $-OCOR^{63C}$, (k) $-NR^{64C}SO_2R^{65C}$, (l) $-NR^{66C}COOR^{67C}$, (m) $-NR^{68C}CONR^{69C}R^{70C}$, (n) -B $(OR^{71C})_2$, (o) - $SO_2R^{72C}$ and (p) $-N(SO_2R^{72C})_2$.),

$R^{52C}$-$R^{62C}$, $R^{64C}$, $R^{66C}$ and $R^{68C}$-$R^{71C}$ are, each independently,

(1) hydrogen,
(2) C1-8 alkyl,
(3) C2-8 alkenyl,
(4) C2-8 alkynyl,
(5) $Cyc2^C$ or
(6) C1-8 alkyl, C2-8 alkenyl or C2-8 alkynyl substituted by $Cyc2^C$, $-OR^{73C}$, $-COOR^{74C}$ or $-NR^{75C}R^{76C}$,

$R^{57C}$ and $R^{58C}$, $R^{61C}$ and $R^{62C}$, or $R^{69C}$ and $R^{70C}$, taken together, are

(1) C2-6 alkylene,
(2) -(C2-6 alkylene)-O-(C2-6 alkylene)-,
(3) -(C2-6 alkylone)-S-(C2-6 alkylene)- or
(4) -(C2-6 alkylene)-$NR^{197C}$-(C2-6 alkylene)- (wherein $R^{197C}$ is hydrogen, C1-8 alkyl, phenyl or C1-8 alkyl substituted by phenyl.),

$R^{63C}$, $R^{65C}$, $R^{67C}$ and $R^{72C}$ are, each independently,

(1) C1-8 alkyl,
(2) C2-8 alkenyl,
(3) C2-8 alkynyl,
(4) $Cyc2^C$ or
(5) C1-8 alkyl, C2-8 alkenyl or C2-8 alkynyl substituted by $Cyc2^C$, $-OR^{73C}$, $-COOR^{74C}$ or $-NR^{75C}R^{76C}$,

$R^{73C}$-$R^{76C}$ are, each independently, hydrogen, C1-8 alkyl, $Cyc2^C$ or C1-8 alkyl substituted by $Cyc2^C$,
$Cyc2^C$ has the same meaning as Cycl. With the proviso that, $Cyc2^C$ may be optionally substiututed by 1-5 of $R^{77C}$,
$R^{77C}$ is,

(1) C1-8 alkyl,
(2) halogen,
(3) nitro,
(4) trifluoromethyl,
(5) trifluoromethoxy,
(6) nitrile,
(7) $-OR^{78C}$,
(8) $-NR^{79C}R^{80C}$,
(9) $-COOR^{81C}$,
(10) $-SR^{82C}$,
(11) $-CONR^{83C}R^{84C}$,
(12) C2-8 alkenyl,
(13) C2-8 alkynyl,
(14) keto,
(15) $Cyc6^C$,

(16) $-NR^{161C}COR^{162C}$,
(17) $-SO_2NR^{163C}R^{164C}$,
(18) $-OCOR^{165C}$,
(19) $-NR^{166C}SO_2R^{167C}$,
(20) $-NR^{168C}COOR^{169C}$,
(21) $-NR^{170C}CONR^{171C}R^{172C}$,
(22) $-SO_2R^{173C}$,
(23) $-N(SO_2R^{167C})_2$,
(24) $-S(O)R^{173C}$ or
(25) C1-8 alkyl, C2-8 alkenyl or C2-8 alkynyl substituted by 1-5 of optionally selected from (a) halogen, (b) $-OR^{78C}$, (c) $-NR^{79C}R^{80C}$, (d) $-COOR^{81C}$, (e) - $SR^{82C}$, (f) $-CONR^{83C}R^{84C}$, (g) keto, (h) $Cyc6^C$, (i) $-NR^{161C}COR^{162C}$, (j) $-SO_2NR^{163C}R^{164C}$, (k) $-OCOR^{165C}$, (l) $-NR^{166C}SO_2R^{167C}$, (m) $-NR^{168C}COOR^{169C}$, (n) -$NR^{170C}CONR^{171C}R^{172C}$, (o) $-SO_2R^{173C}$, (p) $-N(SO_2R^{167C})_2$ and (q) $-S(O)R^{173C}$,

$R^{78C}$-$R^{84C}$, $R^{161C}$-$R^{164C}$, $R^{166C}$, $R^{168C}$ and $R^{170C}$-$R^{172C}$ are, each independently, (a) hydrogen, (b) C1-8 alkyl, (c) C2-8 alkenyl, (d) C2-8 alkynyl, (e) $Cyc6^C$, (f) C1-8 alkyl, C2-8 alkenyl or C2-8 alkynyl substituted by $Cyc6^C$, $-OR^{174C}$, $-COOR^{175C}$, - $NR^{176C}R^{177C}$ or $-CONR^{178C}R^{179C}$,
$R^{83C}$ and $R^{84C}$, $R^{163C}$ and $R^{164C}$, or $R^{171C}$ and $R^{172C}$, taken together, are

(1) C2-6 alkylene,
(2) -(C2-6 alkylene)-O-(C2-6 alkylene)-,
(3) -(C2-6 alkylene)-S-(C2-6 alkylene)- or
(4) -(C2-6 alkylene)-$NR^{198C}$-(C2-6 alkylene)- (wherein $R^{198C}$ is hydrogen, C1-8 alkyl, phenyl or C1-8 alkyl substituted by phenyl.),

$R^{165C}$, $R^{167C}$, $R^{169C}$ and $R^{173C}$ are, each independently, (a) C1-8 alkyl, (b) C2-8 alkenyl, (c) C2-8 alkynyl, (d) $Cyc6^C$ or (e) C1-8 alkyl, C2-8 alkenyl or C2-8 alkynyl substituted by $Cyc6^C$, $-OR^{174C}$, $-COOR^{175C}$, $-NR^{176C}R^{177C}$ or $-CONR^{178C}R^{179C}$,
$R^{174C}$-$R^{177C}$ are, each independently,

(1) hydrogen,
(2) C1-8 alkyl,
(3) $Cyc6^C$ or
(4) C1-8 alkyl substituted by $Cyc6^C$,

$R^{178C}$ and $R^{179C}$, taken together, are

(1) C2-6 alkylene,
(2) -(C2-6 alkylene)-O-(C2-6 alkylene)-,
(3) -(C2-6 alkylene)-S-(C2-6 alkylene)- or
(4) -(C2-6 alkylene)-$NR^{199C}$-(C2-6 alkylene)- (wherein $R^{199C}$ is hydrogen, C1-8 alkyl, phenyl or C1-8 alkyl substituted by phenyl.),

$Cyc6^C$ is C3-8 mono-carbocyclic ring or 3-8 membered mono-cyclic hetero ring containing 1-4 nitrogen atom(s), 1-2 oxygen atom(s) and/or 1-2 sulfur atom(s). With the proviso that, $Cyc6^C$ may be optionally substituted by 1-5 of $R^{180C}$,
$R^{180C}$ is,

(1) C1-8 alkyl,
(2) halogen,
(3) nitro,
(4) trifluoromethyl,
(5) trifluoromethoxy,
(6) nitrile,
(7) $-OR^{181C}$,
(8) $-NR^{182C}R^{183C}$,
(9) $-COOR^{184C}$,
(10) $-SR^{185C}$ or

(11) $-CONR^{186C}R^{187C}$,

$R^{181C}$-$R^{187C}$ are, each independently,

(1) hydrogen,
(2) C1-8 alkyl,
(3) phenyl or
(4) C1-8 alkyl substituted by phenyl,

$R^{182C}$ and $R^{183C}$, or $R^{186C}$ and $R^{187C}$, taken together, are

(1) C2-6 alkylene,
(2) -(C2-6 alkylene)-O-(C2-6 alkylene)-,
(3) -(C2-6 alkylene)-S-(C2-6 alkylene)- or
(4) -(C2-6 alkylene)-$NR^{200C}$-(C2-6 alkylene)- (wherein $R^{200C}$ is hydrogen, C1-8 alkyl, phenyl, C1-8 alkyl substituted by phenyl.),

$R^{2C}$ is,

(1) hydrogen,
(2) C1-8 alkyl,
(3) C2-8 alkenyl,
(4) C2-8 alkynyl,
(5) $-OR^{90C}$,
(6) $Cyc3^{C}$ or
(7) C1-8 alkyl, C2-8 alkenyl or C2-8 alkynyl substituted by 1-5 of optionally selected from (a) halogen, (b) $-OR^{90C}$, (c) $-SR^{91C}$, (d) $-NR^{92C}R^{93C}$, (e) - $COOR^{94C}$, (f) $-CONR^{95C}R^{96C}$, (g) $-NR^{97C}COR^{98C}$, (h) $-SO_2NR^{99C}R^{100C}$, (i) $-OCOR^{101C}$, (j) $-NR^{102C}SO_2R^{103C}$, (k) $-NR^{104C}COOR^{105C}$, (l) $-NR^{106C}CONR^{107C}R^{108C}$, (m) $Cyc3^{C}$, (n) keto and (o) $-N(SO_2R^{103C})_2$,

$R^{90C}$-$R^{100C}$, $R^{102C}$, $R^{104C}$ and $R^{106C}$-$R^{108C}$ are, each independently,

(1) hydrogen,
(2) C1-8 alkyl,
(3) C2-8 alkenyl,
(4) C2-8 alkynyl,
(5) $Cyc3^{C}$ or
(6) C1-8 alkyl, C2-8 alkenyl or C2-8 alkynyl substituted by $Cyc3^{C}$,

$R^{95C}$ and $R^{96C}$, $R^{99C}$ and $R^{100C}$, or $R^{107C}$ and $R^{108C}$, taken together, are

(1) C2-6 alkylene,
(2) -(C2-6 alkylene)-O-(C2-6 alkylene)-,
(3) -(C2-6 alkylene)-S-(C2-6 alkylene)- or
(4) -(C2-6 alkylene)-$NR^{201C}$-(C2-6 alkylene)-,

$R^{201C}$ is hydrogen, C1-8 alkyl, phenyl or C1-8 alkyl substituted by phenyl,
$R^{101C}$, $R^{103C}$ and $R^{105C}$ are, each independently,

(1) C1-8 alkyl,
(2) C2-8 alkenyl,
(3) C2-8 alkynyl or
(4) $Cyc3^{C}$, or C1-8 alkyl, C2-8 alkenyl or C2-8 alkynyl substituted by $Cyc3^{C}$,

$Cyc3^{C}$ has the same meaning as $Cyc1^{C}$.
With the proviso that, $Cyc3^{C}$ may be optionally substituted by 1-5 of $R^{109C}$,
$R^{109C}$ has the same meaning as $R^{51C}$,
$R^{3C}$ and $R^{4C}$ are, each independently,

(1) hydrogen,
(2) C1-8 alkyl,
(3) C2-8 alkenyl,
(4) C2-8 alkynyl,
(5) $-COOR^{120C}$,
(6) $-CONR^{121C}R^{122C}$,
(7) $Cyc4^{C}$ or
(8) C1-8 alkyl, C2-8 alkenyl or C2-8 alkynyl substituted by 1-5 of selected from (a) halogen, (b) nitrile, (c) $Cyc4^{C}$, (d) $-COOR^{120C}$, (e) $-CONR^{121C}R^{122C}$, (f) $-OR^{123C}$, (g) $-SR^{124C}$, (h) $-NR^{125C}R^{126C}$, (i) $-NR^{127C}COR^{128C}$, (j) $-SO_2NR^{129C}R^{130C}$, (k) $-OCOR^{131C}$, (l) $-NR^{132C}SO_2R^{133C}$, (m) $-NR^{134C}COOR^{135C}$, (n) $-NR^{136C}CONR^{137C}R^{138C}$, (o) $-S-SR^{139C}$, (p) $-NHC(=NH)NHR^{140C}$, (q)keto, (r) $-NR^{145C}CONR^{146C}COR^{147C}$ and (s) $-N(SO_2R^{133C})_2$,

$R^{120C}$-$R^{130C}$, $R^{132C}$, $R^{134C}$, $R^{136C}$-$R^{138C}$, $R^{145C}$ and $R^{146C}$ are, each independently,

(1) hydrogen,
(2) C 1-8 alkyl,
(3) C2-8 alkenyl,
(4) C2-8 alkynyl,
(5) $Cyc4^{C}$ or
(6) C1-8 alkyl, C2-8 alkenyl or C2-8 alkynyl substituted by $Cyc4^{C}$, halogen,$-OR^{148C}$, $-SR^{149C}$, $-COOR^{150C}$ or $-NHCOR^{141C}$,

$R^{121C}$ and $R^{122C}$, $R^{129C}$ and $R^{130C}$, or $R^{137C}$ and $R^{138C}$, taken together, are

(1) C2-6 alkylene,
(2) -(C2-6 alkylene)-O-(C2-6 alkylene)-,
(3) -(C2-6 alkylene)-S-(C2-6 alkylene)- or
(4) -(C2-6 alkylene)-$NR^{202C}$-(C2-6 alkylene)- (wherein $R^{202C}$ is hydrogen, C1-8 alkyl, phenyl, C1-8 alkyl substituted by phenyl.),

$R^{131C}$, $R^{133C}$, $R^{135C}$, $R^{139C}$ and $R^{147C}$ are, each independently,

(1) C1-8 alkyl,
(2) C2-8 alkenyl,
(3) C2-8 alkynyl,
(4) $Cyc4^{c}$ or
(5) C1-8 alkyl, C2-8 alkenyl or C2-8 alkynyl substituted by $Cyc4^{c}$, halogen, $-OR^{148C}$, $-SR^{149C}$, $-COOR^{150C}$ or $-NHCOR^{141C}$,

$R^{140C}$ is hydrogen, $COOR^{142C}$ or $-SO_2R^{143C}$,
$R^{141C}$-$R^{143C}$ are, each independently,

(1) C1-8 alkyl,
(2) C2-8 alkenyl,
(3) C2-8 alkynyl,
(4) $Cyc4^{C}$ or
(5) C1-8 alkyl, C2-8 alkenyl or C2-8 alkynyl substituted by $Cyc4^{C}$,

$R^{148C}$-$R^{150C}$ are, each independently,

(1) hydrogen,
(2) C1-8 alkyl,
(3) C2-8 alkenyl,
(4) C2-8 alkynyl,
(5) $Cyc4^{C}$ or
(6) C1-8 alkyl, C2-8 alkenyl or C2-8 alkynyl substituted by $Cyc4^{C}$,

$Cyc4^C$ has the same meaning as $Cyc1^C$. With the proviso that, $Cyc4^C$ may be optionally substituted by 1-5 of $R^{144C}$, $R^{144C}$ has the same meaning as $R^{51C}$,
$R^{3C}$ and $R^{4C}$, taken together, are

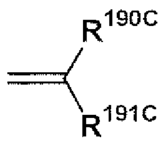

(wherein $R^{190C}$ and $R^{191C}$ are, each independently, the same meaning as $R^{3C}$ or $R^{4C}$.),
$R^{5C}$ is,

(1) hydrogen,
(2) C1-8 alkyl,
(3) $Cyc5^C$ or
(4) C1-8 alkyl substituted by $Cyc5^C$.

(wherein $Cyc5^C$ has the same meaning as $Cyc1^C$. With the proviso that, $Cyc5^C$ may be optionally substituted by 1-5 of $R^{160C}$,
$R^{160C}$ has the same meaning as $R^{51C}$.)]

And also, in *Journal of Medicinal Chemistry*, 43(10), 2049-2063, it is disclosed that the anilide derivatives as CCR5 antagonist, and specifically TAK-779 as developing candidate.

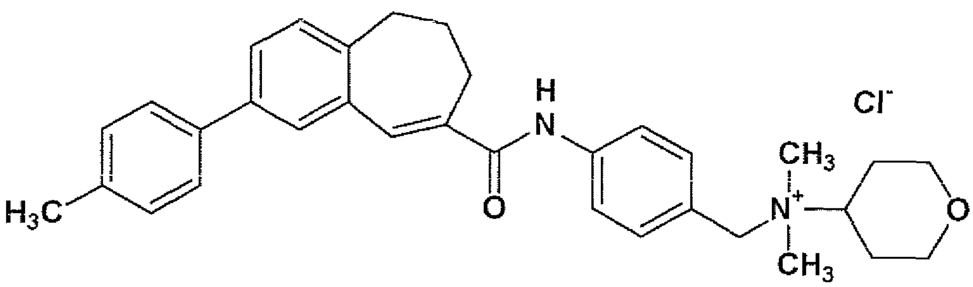

TAK-779

Moreover, in *Journal of Medicinal Chemistry*, 45(14), 3143-3160, it is disclosed that the oximino-piperidino-piperidine amino derivatives as CCR5 antagonist, and specifically SCH-351125 as developing candidate.

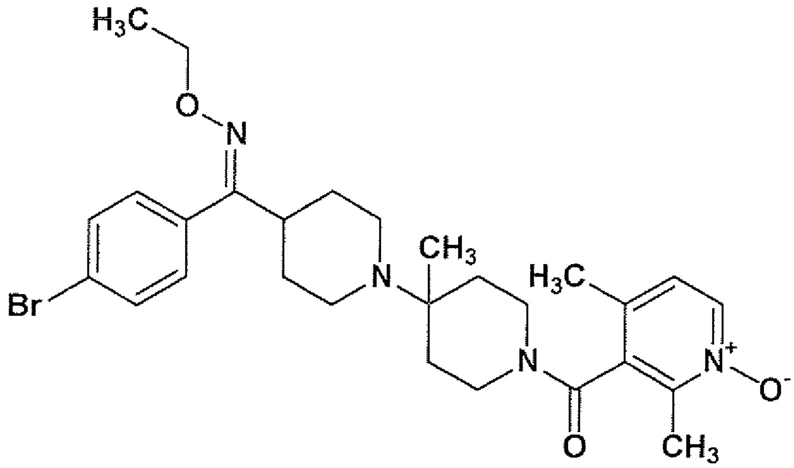

SCH-351125

Disclosure of the Invention

A large number of antagonists and agonists which bind to CCR5 have so far been found. However, the antagonists and the agonists which bind to CCR5 so far found are not clear in terms of their strength to bind to the corresponding receptors. In the present invention, the receptor binding strength not only indicates a simple binding affinity but also means a strength which is concerned in the comprehensive binding including a binding mode with the binding site. In addition, in the present invention, the strong binding site means a substance binding site characterized in that, when a substance is bound thereto, dissociation rate of the substance from a receptor becomes slow, namely high affinity for the receptor, or the occupying space due to binding of the substance to the site and the conformational change added to the receptor become sufficient. The conventional CCR5 binding substances do not bind to such a strong binding site due to weak receptor binding strength, thus posing a problem in that their pharmacological effects are insufficient.

Also, when a usual ligand binding test is used in a screening, it does not bind to the strong binding site in spite of the sufficient ligand binding inhibition activity, so that its pharmacological effect is insufficient which poses a problem in that it cannot sift out antagonists and agonists from which the expected pharmacological effect cannot be expected. Accordingly, concern has been directed toward the development of an efficient method for selecting CCR5-binding antagonists and agonists having sufficient pharmacological effects.

When a method for efficiently finding a compound which has the above characteristics and binds to a strong binding site can be constructed, it becomes possible to find a compound having such a characteristic that a sufficient pharmacological effect can be expected therefrom.

For example, in the case of an anti-HIV action as one of the pharmacological effects of CCR5 antagonists and agonists, when they have a characteristic to bind to the strong binding site, a desirable characteristic in that the dissociation rate is as slow as possible and the region where the gp120 of HIV uses for its binding is occupied as large as possible, or a structure in which a conformational change of the receptor does not fit to the binding of gp120 of HTV is formed, is added in addition to the high binding affinity for the receptor CCR5, which becomes a great advantage. In addition to the general idea on the presence of a strong binding site, when taking note of the anti-HIV activity, the presence of a second loop on CCR5 has been suggested (Lee, B *et al., J. Biol. Chem.*, 274, 9617-26 (1997), Kuhmann, S *et al., J. Virol.*, 71, 8642-8656 (1997), Ross, T *et al., J. Virol.*, 72, 1918 - 1924 (1997)).

However, the presence of a strong binding site on CCR5 for antagonist and agonist and a compound which binds thereto have not been found, and a method for screening the compound has not been known yet.

The present inventors have conducted intensive studies in order to solve the above problems, and found, as a result, that a strong binding site is present on CCR5, and by illustratively constructing a method for screening a compound which binds to the site, further found that the compound is useful, thus resulting in the accomplishment of the present invention.

The present invention relates to:

1. An antagonist or an agonist which binds to a strong binding site of CCR5, excluding the compounds described in WO01/40227, WO02/74769 and WO02/74770 and SCH-351125;
2. A preventive and/or therapeutic agent for an allergic disease, an inflammatory disease, an immune disease

and/or a cancer, which comprises the antagonist or the agonist according to above 1;
3. The preventive and/or therapeutic agent according to above 2, wherein the allergic disease, the inflammatory disease, the immune disease and the cancer are diseases selected from asthma, atopic dermatitis, nettle rash, allergic bronchopulmonary aspergillosis, allergic eosinophilic gastroenteritis, nephritis, nephropathy, hepatitis, arthritis, rheumatoid arthritis, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplanted organ rejection reaction, immunosuppression, cancer metastasis, HIV infection and acquired immunodeficiency syndrome;
4. The preventive and/or therapeutic agent according to above 2, wherein the immune disease is HIV infection, acquired immunodeficiency syndrome and/or transplanted organ rejection reaction;
5. A method for screening a compound which binds to a strong binding site of CCR5, which comprises

(a) allowing a CCR5 expressing cell or a membrane fraction thereof to contact with a compound to be tested,
(b) washing the cell or the membrane from 1 to 12 times, and
(c) adding a labeled ligand and measuring amount of the bound labeled ligand;

6. The method according to above 5, wherein the cell or the membrane is washed from 6 to 10 times;
7. A method for screening a compound which binds to a strong binding site of CCR5, which comprises

(a) allowing a CCR5 expressing cell or a membrane fraction thereof to contact with a compound to be tested,
(b) allowing the test compound-bound cell or a membrane fraction thereof prepared in the above (a) to contact with a labeled anti-CCR5 antibody, and
(c) measuring the labeled anti-CCR5 antibody bound to the CCR5 expressing cell or a membrane fraction thereof;

8. The method according to above 7, wherein the anti-CCR5 antibody is 45531.111 antibody and/or 45523.111 antibody;
9. A method for measuring an occupying ratio of a compound bound to a CCR5 expressing cell or a membrane fraction thereof, which comprises

(a) allowing a CCR5 expressing cell or a membrane fraction thereof to contact with a compound to be tested,
(b) allowing the test compound-bound cell or a membrane fraction thereof prepared in the above step (a) to contact with a labeled anti-CCR5 antibody, and
(c) calculating an occupying ratio of the compound bound to CCR5 on the cell or membrane fraction thereof, based on the ratio of a bound amount of the anti-CCR5 antibody when the compound is bound to CCR5 to a bound amount of the anti-CCR5 antibody when the compound is not bound to CCR5, which is defined as 100%;

10. A method for periodically monitoring an occupying ratio of a compound which binds to a binding site on a CCR5 expressing cell in blood, which comprises

(a) administering a compound which binds to a binding site of CCR5 to a mammal and then separating a cell population containing a cell expressing CCR5 in blood,
(b) allowing the separated cell population to contact with a labeled anti-CCR5 antibody, and
(c) calculating an occupying ratio of the compound which binds to a binding site of CCR5 on the separated cell, based on the ratio of a bound amount of the anti-CCR5 antibody when the compound is bound to CCR5 to a bound amount of the anti-CCR5 antibody when the compound is not bound to CCR5, which is defined as 100%;

11. The method according to above 10, wherein the anti-CCR5 antibody is 45531.111 antibody and/or 45523.111 antibody;
12. A method for determining a dose and an administration frequency which show such an efficacy that an inhibition ratio of about 50% or 90% can be obtained in administering a compound which binds to a binding site of CCR5, which comprises:

(a) measuring IC50 value or IC90 value of the inhibitory activity of a compound which binds to a binding site of CCR5, by an in vitro activity measuring method,
(b) calculating an occupying ratio of a compound which binds to a binding site of CCR5 on a CCR5 expressing cell at a compound concentration corresponding to the IC50 value or IC90 value described in the above (a),

by the method according to above 9, and
(c) comparing the occupying ratio of a compound which binds to a binding site of CCR5, obtained at each dose and monitoring time by the monitoring method according to above 10, with the occupying ratio obtained by the method described in the above (b);

13. The method according to above 12, wherein the in vitro activity measuring method is an anti-HIV activity measuring method,
14. A preventive and/or therapeutic agent for an allergic disease, an inflammatory disease, an immune disease and/or a cancer, which comprises a compound which binds to a strong binding site of CCR5 selected by the method according to any one of above 5, 7, 9, 10 and 12 as an active ingredient;
15. The preventive and/or therapeutic agent according to above 14, wherein the immune disease is HIV infection, acquired immunodeficiency syndrome and/or transplanted organ rejection reaction;
16. The preventive and/or therapeutic agent according to above 2 or 14, wherein the administration method is to administer it orally or parenterally at intervals of one day, two days, three days or several days;
17. The preventive and/or therapeutic agent according to above 16, wherein the immune disease is HIV infection, acquired immunodeficiency syndrome and/or transplanted organ rejection reaction;
18. A method for screening an antagonist or an agonist which binds to a strong binding site of CCR5, which comprises administering it orally or parenterally at intervals of one day, two days, three days or several days;
19. An antagonist or an agonist which binds to a strong binding site of a chemokine receptor;
20. A preventive and/or therapeutic agent for an allergic disease, an inflammatory disease, an immune disease and/or a cancer, which comprises the antagonist or the agonist according to above 19;
21. The preventive and/or therapeutic agent according to above 20, wherein the allergic disease, inflammatory disease, immune disease and cancer are diseases selected from asthma, atopic dermatitis, nettle rash, allergic bronchopulmonary aspergillosis, allergic eosinophilic gastroenteritis, nephritis, nephropathy, hepatitis, arthritis, rheumatoid arthritis, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplanted organ rejection reaction, immunosuppression, cancer metastasis, HIV infection and acquired immunodeficiency syndrome;
22. A method for screening a compound which binds to a strong binding site of a chemokine receptor, which comprises

(a) allowing a chemokine receptor expressing cell or a membrane fraction thereof to contact with a compound to be tested,
(b) washing the cell or the membrane from 1 to 12 times, and
(c) adding a labeled ligand and measuring an amount of the bound labeled ligand;

23. A method for screening a compound which binds to a strong binding site of a chemokine receptor, which comprises

(a) allowing a chemokine receptor expressing cell or a membrane fraction thereof to contact with a compound to be tested,
(b) allowing the test compound-bound cell or a membrane fraction thereof prepared in the above step (a) to contact with a labeled anti-chemokine receptor antibody, and
(c) measuring the labeled anti-chemokine receptor antibody bound to the chemokine receptor expressing cell or a membrane fraction thereof;

24. A preventive and/or therapeutic agent for an allergic disease, an inflammatory disease, an immune disease and/or a cancer, which comprises a compound which binds to a strong binding site of a chemokine receptor selected by the method according to above 22 or 23 as an active ingredient;
25. A method for preventing and/or treating a CCR5 intervening disease in a mammal, which comprises administering an effective amount of the agonist or the antagonist according to above 1;
26. A method for preventing and/or treating a chemokine receptor intervening disease in a mammal, which comprises administering an effective amount of the antagonist or the agonist according to above 19;
27. A method for preventing and/or treating an allergic disease, an inflammatory disease, an immune disease and/or a cancer, which comprises administering a compound which binds to a strong binding site of CCR5 selected by the method according to any one of above 5, 7, 9, 10 and 12 as an active ingredient;
28. Use of the agonist and/or the antagonist according to above 1 for producing a preventive and/or therapeutic agent for a CCR5 intervening disease;

29. Use of the agonist and/or the antagonist according to above 19 for producing a preventive and/or therapeutic agent for a chemokine receptor intervening disease;

30. Use of a compound which binds to a strong binding site of CCR5, which is selected by the method according to any one of above 5, 7, 9, 10 and 12, for producing a preventive and/or therapeutic agent for a CCR5 intervening disease;

31. A compound which binds to a strong binding site of CCR5 selected by the method according to any one of above 5, 7, 9, 10 and 12; and

32. A method for preparation of compound which binds to a strong binding site of CCR5 selected by the method according to any one of above 5, 7, 9, 10 and 12.

In the present invention, the antagonist or the agonist which binds to a strong binding site of CCR5 or chemokine receptor is a substance having such characteristics that, when said substance is bound thereto, dissociation rate of the substance from the receptor becomes slow, namely high affinity for the receptor, or the occupying space due to binding of the substance to the site and the conformational change added to the receptor become sufficient.

In the present invention, the diseases which can be prevented and/or treated by the use of an antagonist or an agonist which binds to a strong binding site of CCR5 may be all diseases in which CCR5 is concerned, and are not limited to an allergic disease, an inflammatory disease, an immune disease and a cancer. Examples of the allergic disease, inflammatory disease, immune disease and cancer include asthma, atopic dermatitis, nettle rash, allergic bronchopulmonary aspergillosis, allergic eosinophilic gastroenteritis, nephritis, nephropathy, hepatitis, arthritis, rheumatoid arthritis, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplanted organ rejection reaction, immunosuppression, cancer metastasis, HIV infection and acquired immunodeficiency syndrome and the like.

In the present invention, the diseases which can be prevented and/or treated by the use of an antagonist or an agonist which binds to a strong binding site of a chemokine receptor may be all diseases in which the chemokine receptor is concerned, and are not limited to an allergic disease, an inflammatory disease, an immune disease and a cancer. Examples of the allergic disease, inflammatory disease, immune disease and cancer include asthma, atopic dermatitis, nettle rash, allergic bronchopulmonary aspergillosis, allergic eosinophilic gastroenteritis, nephritis, nephropathy, hepatitis, arthritis, rheumatoid arthritis, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplanted organ rejection reaction, immunosuppression, cancer metastasis, HIV infection and acquired immunodeficiency syndrome and the like.

In the present invention, the antagonist and the agonist which bind to a strong binding site of CCR5 include all of those which have so far been found and which will be found in the future.

In the present invention, the antagonist and the agonist which bind to a strong binding site of a chemokine receptor include all of those which have so far been found and which will be found in the future.

In the present invention, the compound means an antagonist or an agonist.

In the present invention, the site to which the compound is bound is not limited to CCR5. For example, it can also be applied to a compound (an agonist and/or an antagonist) which binds to a general chemokine receptor, namely to a strong binding site of a chemokine receptor. Examples of the chemokine receptor include CCR1, CCR2, CCR3, CCR4, CCR6, CCR7, CCR8, CCR9, CCR10, CCR11, CXCR1, CXCR2, CXCR3, CXCR4, CXCR5, CXCR6, CX3CR, XCR1 and the like.

Also, regarding the screening method of the present invention, it can also be carried out using a membrane fraction of CCR5 instead of a CCR5 expressing cell. In addition, it can also be carried out using other chemokine receptor expressing cell or a membrane fraction thereof, instead of CCR5 expressing cell or a membrane fraction thereof.

Among the present inventions, regarding the method which uses an anti-CCR5 antibody, it can also be carried out using other chemokine receptor antibody instead of an anti-CCR5 antibody. For example, when it is an antibody for other chemokine receptor antibody, and an antagonist or an agonist is bound to the chemokine receptor, it may be any substance which has a property of not binding to the chemokine receptor.

The anti-CCR5 antibody to be used in the screening of the present invention is not limited to 45531.111 and 45523.111, and it may be any antibody which does not bind to CCR5 when an agonist or an antagonist which binds to a strong binding site is bound to CCR5.

The labeling to be employed in the screening of the present invention may be any means which can identify bound amount of a substance, and its examples include radiation labeling, fluorescence labeling, enzyme labeling and the like, though not limited thereto.

Regarding the labeled ligand to be used in the present invention, it is preferably a radioactive ligand (e.g., a ligand labeled with an isotope such as $^{3}H$, $^{14}C$, $^{32}P$, $^{35}S$ or $^{125}I$), and more preferably a substance in which a ligand of

CCR5 or a chemokine receptor is iodine-labeled ($^{125}I$). Regarding the ligand of CCR5 or a chemokine receptor, MIP-1α, MIP-1β, RANTES, MCP-1, SDF-1, MDC, TARC, MIP-3α, MIP-3β, SLC, Eotaxin, CTACK, I-309, TECK, CCL28, IL-8, GRO, IP-10, BCA, CXCL, Fractalkine, lymphotactin and the like can be exemplified.

Included in the mammal in the present invention are human and mammals other than human (e.g., rat, mouse, guinea pig, rabbit, sheep, swine, bovine, cat, dog, monkey, *etc.*).

In the present invention, the term several days means from 4 to 14 days.

Whether the compound of the present invention which binds to a strong binding site of CCR5 is an antagonist or an agonist can be judged, for example, by a method which is described later. This judging method is merely a method which can judge whether it is an antagonist or an agonist, and it can be judged by any conventionally known method other than the method which is described later.

The aforementioned judging method of antagonist and agonist may be carried out before or after the screening of the compound of the present invention which binds to a strong binding site of CCR5, and its order is not limited.

The screening method of the present invention and the strong binding site are described below in detail.

The present inventors have confirmed the presence of a strong binding site by the following three tests using CCR5 antagonists.

(1) Examination on the association and dissociation of compounds which bind to strong binding site

Inhibitory effects of CCR5 antagonists (e.g., TAK-779, SCH-351125 and triazaspiro[5.5]undecane derivatives) were examined using a binding test system of CCR5 endogenous ligand to CCR5. In this test system, each compound is once allowed to bind to CCR5, treated with washing which is stronger than usual by repeating the washing (e.g., repetition of from 1 to 12 times, preferably from 6 to 10 times, more preferably 8 times or more) in order to remove the compound, and then allowed to stand for a predetermined period of time (e.g., 4 hours or more of standing) in order to generate a new equilibrium state, thereby verifying its inhibitory effect. As a result, SCH-351125 and TAK-779 showed IC50 values of 16.7 and 10.4 nmol/l, respectively. Contrary to this, (3R)-1-butyl-2,5-dioxo-3-((1R)-1-hydroxy-1-cyclohexylmethyl)-9-(4-(4-carboxyphenyloxy)phenylmethyl)-1,4,9-triazaspiro[5.5]undecane hydrochloride (hereinafter referred to as "compound 1") and (3R)-1-butyl-2,5-dioxo-3-((1R)-1-hydroxy-1-cyclohexylmethyl)-9-(4-(4-methylaminocarbonylphenyloxy)phenylmethyl)-1,4,9-triazaspiro[5.5]undecane hydrochloride (hereinafter referred to as "compound 2") showed $IC_{50}$ values of 4.2 and 1.5 nmol/l, respectively, thus finding compounds having strong ligand binding inhibitory effect even after this operation (under severe washing condition).

(2) Examination on the binding mode of compounds which bind to strong binding site

Also, in order to examine binding mode of the compounds having strong ligand binding inhibitory effect in the above test, CCR5 antagonist (e.g., TAK-779, SCH-351125 and triazaspiro[5.5]undecane derivatives) and anti-CCR5 antibodies (45531.111 (R & D Cat No. FAB182F, Lot JG019021) and 45523.111 (R & D Cat No. FAB181F, Lot JG019033)) were used. As a result, TAK-779 inhibited CCR5 binding of both 45531.111 and 45523.111 antibodies only weakly. SCH-351125 inhibited binding of 45523.111, but its inhibition of 45531.111 antibody binding was weak at the similar degree of TAK-779. However, a triazaspiro[5.5]undecane derivative (e.g., compound 1 or compound 2) strongly inhibited binding of both antibodies. That is, it was found that the triazaspiro[5.5]undecane derivatives have a different binding mode for CCR5 in comparison with TAK-779 and SCH-351125. In other words, the triazaspiro[5.5]undecane derivatives (e.g., compound 1 and compound 2) have characteristics in that they have the highest affinity for CCR5 in comparison with the used antibodies and exert influence upon more broad regions on CCR5.

(3) Competition test of compounds which bind to strong binding site

In addition, when whether or not the triazaspiro[5.5]undecane derivatives compete with TAK-779 and SCH-351125 was examined, it was confirmed that the compound 1, for example, competes with TAK-779 and SCH-351125 for CCR5.

Based on the above test result showing that the three triazaspiro[5.5]undecane derivatives compete with TAK-779 and SCH-351125, it was revealed that they are not necessarily bind to completely different sites. However, it was proved by a test using 45531.111 and 45523.111 that these compounds have a site common to the binding site, but three-dimensional structures under a state in which the compounds are bound to CCR5 are different. The triazaspiro [5.5]undecane derivatives are characterized in that they have more strong ligand binding inhibitory effect under severe washing condition, and their inhibition for the binding of antibodies 45531.111 and 45523.111 are also more strong. Based on these facts, a characteristic of the triazaspiro[5.5]undecane derivatives is that they are not limited to the region consisting of the epitope determined by the antibodies 45531.111 and 45523.111, but rather their binding induces a conformational change in CCR5 and thereby exerts influence upon broad regions. By such a change, affinity of a

part where contacted with CCR5 becomes high, or large energy is required for a change from a stable association condition to a dissociation condition, so that the dissociation rate becomes slow.

In the present invention, compounds having a characteristic binding mode such as of the above triazaspiro [5.5]undecane derivatives are defined as antagonists which bind to a strong binding site.

Also, in order to prove usefulness of the antagonists which bind to a strong binding site, relationship between the characteristics of antagonists which bind to a strong binding site and the inhibitory effect on cell migration was examined using CCR5 antagonists (e.g., TAK-779, SCH-351125 and triazaspiro[5.5]undecane derivatives). Regarding the 50% inhibition ratio of cell migration, SCH-351125 was 14.4 nmol/l and TAK-779 was 55 nmol/l, while a triazaspiro [5.5]undecane derivative such as the compound 1 was 4.3 nmol/l. That is, it was found that a compound showing more strong ligand binding inhibitory effect under severe washing condition and antibody binding inhibitory effect using anti-CCR5 antibodies (45531.111 and 45523.111) shows more stronger inhibitory effect against cell migration as one of the pharmacological activities of CCR5 antagonists. In addition, it also correlated with an effect on HIV (anti-HIV activity) which can be regarded also as a ligand of CCR5.

Accordingly, the present inventors have found that the method for screening a compound which binds to a strong binding site can be established by measuring a ligand binding inhibitory activity under a severe washing condition and measuring binding inhibition of either one of 45531.111 antibody and 45523.111 antibody (preferably 45531.111 antibody) or both of the antibodies.

The compound of the present invention which binds to a strong binding site can be judged by the screening in which a ligand binding inhibitory activity is measured under a severe washing condition and/or the screening which uses an anti-CCR5 antibody.

In addition, the present inventors have constructed a method for monitoring binding of these compounds which bind to strong binding site to receptors in actual circulating blood, and confirmed that these compounds are binding thereto for a prolonged period of time. The present inventors have linked the compound in vitro to cells expressing CCR5 and transferred into an animal such as mouse, and the cells were periodically collected to measure the residual compound on CCR5. An antibody whose binding is inhibited by the compound may be used in the measurement of the residual compound, but it is monitored preferably using the above 45531.111 antibody. As a result, it was found that the compound which binds to a strong binding site remains on CCR5 for a prolonged period of time even in the circulation blood. That is, persistence of pharmacological effects for a prolonged period of time can be cited as a characteristic of the compound.

Accordingly, the present inventors have found a new strong binding site of CCR5 which is different from the conventionally known binding site and also found that a compound binding thereto persistently binds thereto, thus resulting in the accomplishment of the present invention.

Since such a compound which binds to a strong binding site can continue its action, administration frequency of a medicament can be reduced. For example, pharmacological effects of CCR5 antagonist and/or agonist are continued even by oral or parenteral administration at intervals of one day, two days, three days or several days.

Application for medicaments:

The CCR5 antagonist and/or the CCR5 agonist of the present invention is useful in animal included human, especially human, as an agent for prevention and/or treatment of various inflammatory diseases, asthma, atopic dermatitis, nettle rash, allergic diseases (allergic bronchopulmonary aspergillosis, allergic eosinophilic gastroenteritis and the like), nephritis, nephropathy, hepatitis, arthritis, chronic rheumatoid arthritis, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune diseases, transplant rejection, immunosuppression, cancer metastasis, acquired immunodeficiency syndrome and the like.

For the purpose above described, the CCR5 antagonist and/or the CCR5 agonist of the present invention may be normally administered systemically or locally, usually by oral or parenteral administration, optionally in combination with other drugs.

The doses to be administered are determined depending upon, for example, age, body weight, symptom, the desired therapeutic effect, the route of administration, and the duration of the treatment. In the human adult, the doses per person are generally from 1 ng to 1000 mg, by oral administration, once per several days, once three days, once two days, once a day, or up to several times per day, and from 1 ng to 100 mg, by parenteral administration (preferably intravenous administration), once per several days, once three days, once two days, once a day, or up to several times per day, or continuous administration from 1 to 24 hours per day into a vein.

As mentioned above, the dosage may be changed due to various conditions or clinical state. Therefore, there are cases in which doses lower than or greater than the ranges specified above may be used.

The CCR5 antagonist and/or the CCR5 agonist of the present invention may be administered in various forms such as solid forms for oral administration, liquid forms for oral administration, or forms for parenteral administration,

for example, injections, drugs for external use, suppositories, eye-drops, inhalations and the like, optionally in combination with other drugs.

Solid forms for oral administration include compressed tablets, pills, capsules, powdered drugs, and granules. Capsules include hard capsules and soft capsules. And compressed tablets include sublingual tablets, buccal adhesive tablets, and rapidly-disintegrating buccal tablets *etc*.

In such solid forms, one or more of the active compound(s) may be admixed with excipients (such as lactose, mannitol, glucose, microcrystalline cellulose or starch), binders (such as hydroxypropyl cellulose, polyvinylpyrrolidone or magnesium aluminometasilicate), disintegrants (such as cellulose calcium glycolate), lubricants (such as magnesium stearate), stabilizing agents, and solubilization auxiliary agents (such as glutamic acid or aspartic acid) and prepared according to methods well known in normal pharmaceutical practice. And also, the solid forms may, if desired, be coated with coating agents (such as sugar, gelatin, hydroxypropyl cellulose or hydroxypropylmethyl cellulose phthalate), or be coated with two or more films. And further, coating may include containment within capsules of absorbable materials such as gelatin.

The sublingual tablets are produced in accordance with a conventionally known method. For example, they are used by mixing one or more active substances with an excipient (lactose, mannitol, glucose, microcrystalline cellulose, colloidal silica, starch, *etc*.), a binder (hydroxypropyl cellulose, polyvinyl pyrrolidone, magnesium aluminometasilicate, *etc*.), a disintegrating agent (starch, L-hydroxypropyl cellulose, carboxymethyl cellulose, croscarmellose sodium, calcium cellulose glycolate, *etc*.), a lubricant (magnesium stearate, *etc*.), a swelling agent (hydroxypropyl cellulose, hydroxypropylmethyl cellulose, carbopol, carboxymethyl cellulose, polyvinyl alcohol, xanthan gum, guar gum , *etc*.), a swelling auxiliary agent (glucose, fructose, mannitol, xylitol, erythritol, maltose, trehalose, phosphate, citrate, silicate, glycine, glutamic acid, arginine, *etc*.), a stabilizer, a solubilization auxiliary agent (polyethylene glycol, propylene glycol, glutamic acid, aspartic acid, *etc*.), a flavor (orange, strawberry, mint, lemon, vanilla, *etc*.) and the like, and making the mixture into tablets in accordance with the usual way. Also, if necessary, they may be coated with a coating agent (sucrose, gelatin, hydroxypropyl cellulose, hydroxypropylmethyl cellulose phthalate, *etc*.) or may be coated with two or more layers. In addition, generally used additives, such as antiseptics, antioxidants, coloring agents and sweeteners, can be added thereto, if necessary.

The buccal adhesive tablets are produced in accordance with a conventionally known method. For example, they are used by mixing one or more active substances with an excipient (lactose, mannitol, glucose, microcrystalline cellulose, colloidal silica, starch, *etc*.), a binder (hydroxypropyl cellulose, polyvinyl pyrrolidone, magnesium aluminometasilicate, *etc*.), a disintegrating agent (starch, L-hydroxypropyl cellulose, carboxymethyl cellulose, croscarmellose sodium, calcium cellulose glycolate, *etc*.), a lubricant (magnesium stearate, *etc*.), an adhesive agent (hydroxypropyl cellulose, hydroxypropylmethyl cellulose, carbopol, carboxymethyl cellulose, polyvinyl alcohol, xanthan gum, guar gum, *etc*.), an adhesion auxiliary agent (glucose, fructose, mannitol, xylitol, erythritol, maltose, trehalose, phosphate, citrate, silicate, glycine, glutamic acid, arginine, *etc*.), a stabilizer, a solubilization auxiliary agent (polyethylene glycol, propylene glycol, glutamic acid, aspartic acid, *etc*.), a flavor (orange, strawberry, mint, lemon, vanilla, *etc*.) and the like, and making the mixture into tablets in accordance with the usual way. Also, if necessary, they may be coated with a coating agent (sucrose, gelatin, hydroxypropyl cellulose, hydroxypropylmethyl cellulose phthalate, *etc*.) or may be coated with two or more layers. In addition, generally used additives, such as antiseptics, antioxidants, coloring agents and sweeteners, can be added thereto, if necessary.

The rapidly-disintegrating buccal tablets are produced in accordance with a conventionally known method. For example, they are used by mixing one or more active substances as such, or active substances prepared by coating stock powders or granulated stock particles using an appropriate coating agent (ethyl cellulose, hydroxypropyl cellulose, hydroxypropylmethyl cellulose, an acrylic acid/methacrylic acid copolymer, *etc*.) and a plasticizer (polyethylene glycol, triethyl citrate, *etc*.), with an excipient (lactose, mannitol, glucose, microcrystalline cellulose, colloidal silica, starch, *etc*.), a binder (hydroxypropyl cellulose, polyvinyl pyrrolidone, magnesium aluminometasilicate, *etc*.), a disintegrating agent (starch, L-hydroxypropyl cellulose, carboxymethyl cellulose, croscarmellose sodium, calcium cellulose glycolate, *etc*.), a lubricant (magnesium stearate, *etc*.), a dispersion auxiliary agent (glucose, fructose, mannitol, xylitol, erythritol, maltose, trehalose, phosphate, citrate, silicate, glycine, glutamic acid, arginine, *etc*.), a stabilizer, a solubilization auxiliary agent (polyethylene glycol, propylene glycol, glutamic acid, aspartic acid, *etc*.), a flavor (orange, strawberry, mint, lemon, vanilla, *etc*.) and the like, and making the mixture into tablets in accordance with the usual way. Also, if necessary, they may be coated with a coating agent (sucrose, gelatin, hydroxypropyl cellulose, hydroxypropylmethyl cellulose phthalate, *etc*.) or coated with two or more layers. In addition, generally used additives, such as antiseptics, antioxidants, coloring agents and sweeteners, can be added thereto, if necessary.

Liquid forms for oral administration include pharmaceutically acceptable solutions, suspensions, emulsions, syrups and elixirs. In such forms, one or more of the active compound(s) may be dissolved, suspended or emulsified into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Dosage forms of external preparations for parenteral administration include, for example, ointments, gels, creams, fomentations, adhesive preparations, liniments, nebula, inhalants, sprays, aerosols, ophthalmic solutions, nasal drops and the like. These contain one or more active substances and are produced by conventionally known methods or generally used recipe.

The ointments are produced by a conventionally known method or generally used recipe. For example, they are prepared by kneading or melting one or more active substances into a base. The ointment base is selected from those which are conventionally known or generally used. For example, those which are selected from higher fatty acids or higher fatty acid esters (adipic acid, myristic acid, palmitic acid, stearic acid, oleic acid, adipic acid ester, myristic acid ester, palmitic acid ester, a stearic acid ester, oleic acid ester, *etc*.), waxes (beeswax, spermaceti, ceresin, *etc*.), surfactants (polyoxyethylene alkyl ether phosphoric acid ester, *etc*.), higher alcohols (cetanol, stearyl alcohol, cetostearyl alcohol, *etc*.), silicon oil (dimethyl polysiloxane, *etc*.), hydrocarbons (hydrophilic petrolatum, white petrolatum, purified lanolin, liquid paraffin, *etc*.), glycols (ethylene glycol, diethylene glycol, propylene glycol, polyethylene glycol, macrogol, *etc*.), plant oil (castor oil, olive oil, sesame oil, turpentine oil, *etc*.), animal oil (mink oil, egg yolk oil, squalane, squalene, *etc*.), water, an absorption accelerator and a rash preventing agent are used alone or as a mixture of two or more. They may further contain moisture keeping agents, preservatives, stabilizers, antioxidants, flavors and the like.

The gels are produced by a conventionally known method or generally used recipe. For example, they are prepared by melting one or more active substances in a base. The gel base is selected from those which are conventionally known or generally used. For example, those which are selected from lower alcohols (ethanol, isopropyl alcohol, *etc*.), gelling agents (carboxymethyl cellulose, hydroxyethyl cellulose, hydroxypropyl cellulose, ethyl cellulose, *etc*.), neutralizing agents (triethanolamine, diisopropanolamine, *etc*.), surfactants (polyethylene glycol monostearate, *etc*.), gums, water, an absorption accelerator and a rash preventing agent are used alone or as a mixture of two or more. They may further contain preservatives, antioxidants, flavors and the like.

The creams are produced by a conventionally known method or generally used recipe. For example, they are prepared by melting or emulsifying one or more active substances in a base. The cream base is selected from those which are conventionally known or generally used. For example, those which are selected from higher fatty acid esters, lower alcohols, hydrocarbons, polyhydric alcohols (propylene glycol, 1,3-butylene glycol, *etc*.), higher alcohols (2-hexyldecanol, cetanol, *etc*.), emulsifying agents (polyoxyethylene alkyl ethers, fatty acid esters, *etc*.), water, an absorption accelerator and a rash preventing agent are used alone or as a mixture of two or more. They may further contain preservatives, antioxidants, flavors and the like.

The fomentations are produced by a conventionally known method or generally used recipe. For example, they are produced by melting one or more active substances in a base, and spreading and coating the kneaded product on a support. The fomentation base is selected from those which are conventionally known or generally used. For example, those which are selected from thickeners (polyacrylic acid, polyvinyl pyrrolidone, gum arabic, starch, gelatin, methyl cellulose, *etc*.), moisture keeping agents (urea, glycerol, propylene glycol, *etc*.), excipients (kaolin, zinc oxide, talc, calcium, magnesium, *etc*.), water, a solubilization auxiliary agent, a tackifier and a rash preventing agent are used alone or as a mixture of two or more. They may further contain preservatives, antioxidants, flavors and the like.

The adhesive preparations are produced by a conventionally known method or generally used recipe. For example, they are produced by melting one or more active substances in a base and spreading and coating on a support. The base for adhesive preparations is selected from those which are conventionally known or generally used. For example, those which are selected from polymer bases, oils and fats, higher fatty acids, tackifiers and rash preventing agents are used alone or as a mixture of two or more. They may further contain preservatives, antioxidants, flavors and the like.

The liniments are produced by a conventionally known method or generally used recipe. For example, they are prepared by dissolving, suspending or emulsifying one or more active substances in one or two or more media selected from water, alcohol (ethanol, polyethylene glycol, *etc*.), higher fatty acid, glycerol, soap, an emulsifying agent, an suspending agent and the like. They may further contain preservatives, antioxidants, flavors and the like.

The nebula, inhalants, and sprays may comprise additional substances other than diluents, such as stabilizing agents, such as sodium hydrogen sulfite, isotonic buffers, such as sodium chloride, sodium citrate or citric acid. For preparation of such sprays, for example, the method described in the United States Patent No. 2,868,691 or 3,095,355 may be used.

Injections for parenteral administration include sterile solutions, suspensions, emulsions and solid forms which are dissolved or suspended into solvent(s) for injection immediately before use. In injections, one or more of the active compound(s) may be dissolved, suspended or emulsified into solvent(s). The solvents may include distilled water for injection, saline, vegetable oil, propylene glycol, polyethylene glycol, alcohol such as ethanol, or a mixture thereof. Injections may comprise some additives, such as stabilizing agents, solubilization auxiliary agents (such as glutamic acid, aspartic acid or POLYSORBATE80 (registered trade mark)), suspending agents, emulsifying agents, soothing agent, buffering agents, preservatives. They may be sterilized at a final step, or may be prepared according to sterile methods. They may also be manufactured in the form of sterile solid forms such as freeze-dried products, which may

be dissolved in sterile water or some other sterile diluent(s) for injection immediately before use.

The ophthalmic solutions for parenteral administration include eye drops, suspension type eye drops, emulsion type eye drops, solid eye drops which are dissolved when used and eye ointments.

These ophthalmic solutions are produced in accordance with conventionally known methods. For example, one or more of the active compound(s) may be dissolved, suspended or emulsified into solvent(s). For example, as the solvent of ophthalmic solutions, sterilized purified water, physiological saline, other aqueous solvent or a non-aqueous solvent for injection (e.g., plant oil, *etc.*) and the like and a combination thereof are used. As occasion demands, the ophthalmic solutions may contain optionally selected tonicity agents (sodium chloride, concentrated glycerol and the like), buffer agents (sodium phosphate, sodium acetate, *etc.*), surfactants (Polysorbate 80 (trade name), Polyoxyl 40 stearate, polyoxyethylene hydrogenated castor oil and the like), stabilizers (sodium citrate, sodium edetate, *etc.*), antiseptics (benzalkonium chloride, parahydroxybenzoate, *etc.*) and the like. These are sterilized in the final step or prepared by an aseptic operation. Alternatively, aseptic solid preparations such as freeze-dried products are produced and used by dissolving in sterilized or sterile purified water or other solvent prior to their use.

As the inhalants for parenteral administration, aerosols, powders for inhalation and solutions for inhalation are included, and the solutions for inhalation may be in such a form that they are used by dissolving or suspending in water or other appropriate solvent when used.

These inhalants are produced in accordance with conventionally known methods.

For example, in the case of solutions for inhalation, they are prepared by optionally selecting, if necessary, antiseptics (benzalkonium chloride, parahydroxybenzoate, *etc.*), coloring agents, buffer agents (sodium phosphate, sodium acetate, *etc.*), tonicity agents (sodium chloride, concentrated glycerol and the like), thickeners (carboxy vinyl polymer, *etc.*), absorption accelerators and the like.

In the case of powders for inhalation, they are prepared by optionally selecting, if necessary, lubricants (stearic acid, a salt thereof, *etc.*), binders (starch, dextrin, *etc.*), excipients (lactose, cellulose, *etc.*), coloring agents, antiseptics (benzalkonium chloride, parahydroxybenzoate, *etc.*), absorption accelerators and the like.

A sprayer (atomizer or nebulizer) is generally used in administering the solutions for inhalation, and an inhalator for powder preparations is generally used in administering the powders for inhalation.

Other examples of the composition for parenteral administration include suppositories for rectal administration and pessaries for vaginal administration prepared by an ordinary formulation comprising one or more active material(s).

The CCR5 antagonist and/or the CCR5 agonist of the present invention may be used together with other drugs, for example, preventive and/or treating agent(s) for HIV infection (particularly an agent for prevention and/or treatment for AIDS). In that case, the drug as such may be mixed with pharmacologically acceptable excipient, binder, disintegrating agent, lubricant, stabilizer, solubilization auxiliary agent, diluent, *etc.* either separately or simultaneously to make into a pharmaceutical preparation and that can be administered either orally or parenterally as a pharmaceutical composition for prevention and/or treatment of HIV infection.

The CCR5 antagonist and/or the CCR5 agonist of the present invention has an infection inhibiting activity to HIV-1 which acquired resistance to other agent for prevention and/or treatment for HIV infection (particularly an agent for prevention and/or treatment for AIDS). Therefore, it is also able to be used for HIV-infected patients to whom other agent for prevention and/or treatment for HIV infection is no longer effective. In that case, although the compound of the present invention may be used solely, it may be also used together with an agent for prevention and/or treatment HIV infection where infected HIV-1 strain acquired resistance or with other drugs.

The present invention includes the case where the CCR5 antagonist and/or the CCR5 agonist of the present invention is combined with a drug which does not inhibit the HIV infection whereby preventing and/or treating effect for HIV infection is enhanced as compared with a single preparation.

Examples of other agent for preventing and/or treating HIV infection used for a combination with the CCR5 antagonist and/or the CCR5 agonist of the present invention are reverse transcriptase inhibitor, protease inhibitor, chemokine antagonist (such as CCR2 antagonist, CCR3 antagonist, CCR4 antagonist, CCR5 antagonist and CXCR4 antagonist), fusion inhibitor, antibody to surface antigen of HIV-1 and vaccine of HIV-1.

Reverse transcriptase inhibitors are concretely (1) nucleic-acid reverse transcriptase inhibitors: zidovudine (brand name: Retrovir), didanosine (brand name: Videx), zalcitabine (brand name: HIVID), stavudine (brand name: Zerit), lamivudine (brand name: Epivir), abacavir (brand name: Ziagen), adefovir, adefovir dipivoxil, emtricitabine (brand name: Coviracil) or PMPA (brand name: Tenofovir) *etc.* and (2) nonnucleic-acid reverse transcriptase inhibitors : nevirapine (brand name: Viramune), delavirdine (brand name: Rescriptor), efavirenz (brand name: Sustiva, Stocklin) or capravirine (AG1549) *etc.*

Protease inhibitors are concretely indinavir (brand name: Crixivan), ritonavir (brand name: Norvir), nelfinavir (brand name: Viracept), saquinavir (brand name: Invirase, Fortovase), amprenavir (brand name: Agenerase), lopinavir (brand name: Kaletra) or tipranavir *etc.*

As chemokine antagonists, internal ligands of chemokine receptor, its derivatives, non-peptide low molecular compounds or antibodies of chemokine receptor are included.

The examples of internal ligand of chemokine receptor are concretely, MIP-1α, MIP-1β, RANTES, SDF-1α, SDF-1β, MCP-1, MCP-2, MCP-4, Eotaxin and MDC *etc*.

The derivatives of internal ligand are concretely, AOP-RANTES, Met-SDF-1α, Met- SDF-1β *etc*.

Antibodies of chemokine receptor are concretely, Pro-140 *etc*.

CCR2 antagonists are concretely written in specification of WO99/07351, WO99/40913, WO00/46195, WO00/46196, WO00/46197, WO00/46198, WO00/46199, WO00/69432 or WO00/69815 or in *Bioorg. Med Chem. Lett.*, 10, 1803 (2000) *etc*.

CCR3 antagonists are concretely written in specification of DE19837386, WO99/55324, WO99/55330, WO00/04003, WO00/27800, WO00/27835, WO00/27843, WO00/29377, WO00/31032, WO00/31033, WO00/34278, WO00/35449, WO00/35451, WO00/35452, WO00/35453, WO00/35454, WO00/35876, WO00/35877, WO00/41685, WO00/51607, WO00/51608, WO00/51609, WO00/51610, WO00/53172, WO00/53600, WO00/58305, WO00/59497, WO00/59498, WO00/59502, WO00/59503, WO00/62814, WO00/73327 or WO01/09088 *etc*.

CCR5 antagonists are concretely written in specification of WO99/17773, WO99/32100, WO00/06085, WO00/06146, WO00/10965, WO00/06153, WO00/21916, WO00/37455, EP1013276, WO00/38680, WO00/39125, WO00/40239, WO00/42045, WO00/53175, WO00/42852, WO00/66551, WO00/66558, WO00/66559, WO00/66141, WO00/68203, JP2000309598, WO00/51607, WO00/51608, WO00/51609, WO00/51610, WO00/56729, WO00/59497, WO00/59498, WO00/59502, WO00/59503, WO00/76933, WO98/25605, WO99/04794, WO99/38514 or in *Bioorg. Med. Chem. Lett.*, 10, 1803 (2000) *etc*.

CXCR4 antagonists are concretely, AMD-3100, T-22, KRH-1120 or the compounds written in specification of WO00/66112 *etc*.

Fusion inhibitors are concretely, T-20 (Pentafuside) and T-1249 *etc*.

The examples of combination agents written above are intended to illustrate, and the present invention is not limited thereto.

The typical examples of the usual dosage in clinical trials of reverse transcriptase inhibitors or protease inhibitors written below are intended to illustrate the present invention, but do not limit them.

| | |
|---|---|
| Zidovudine: | 100 mg capsule, 200 mg per dose, 3 times per day; 300 mg tablet, 300 mg per dose, twice per day; |
| didanosine: | 25-200 mg tablet, 125-200 mg per dose, twice per day; |
| zalcitabine: | 0.375-0.75 mg tablet, 0.75 mg per dose, 3 times per day; |
| stavudine: | 15-40 mg capsule, 30-40 mg per dose, twice per day; |
| lamivudine: | 150 mg tablet, 150 mg per dose, twice per day; |
| abacavir: | 300 mg tablet, 300 mg per dose, twice per day; |
| nevirapine: | 200 mg tablet, 200 mg per dose, once per day for 14 days and then twice per day; |
| delavirdine: | 100 mg tablet, 400 mg per dose, 3 times per day; |
| efavirenz: | 50-200 mg capsule, 600 mg per dose, once per day; |
| indinavir: | 200-400 mg capsule, 800 mg per dose, 3 times per day; |
| ritonavir: | 100 mg capsule, 600 mg per dose, twice per day; |
| nelfinavir: | 250 mg tablet, 750 mg per dose, 3 times per day; |
| saquinavir: | 200 mg capsule, 1200 mg per dose, 3 times per day; |
| amprenavir: | 50-150 mg tablet, 1200 mg per dose, twice per day. |

Brief Description of the Drawings

Fig. 1 shows an inhibitory effect of test compound on binding of anti-hCCR5 antibody to hCCR5 (Example 2).
Fig. 2 shows an *in vivo* binding duration of compound 1 on hCCR5 in mouse (Example 5).

Best Mode for Carrying Out the Invention

The present invention will now be illustrated in detail by way of the following Examples; however the present invention is not limited thereto.

Example 1

Ligand binding inhibition test for CCR5 under severe washing condition:

Human CCR5 expressing cells (hCCR5-CHO cells) were inoculated into a culture plate, cultured using a

medium (Ham's F-12 medium/10% FBS/Antibiotic-Antimycotic) at 37°C for 1 day (or 2 days) in a CO2 incubator of 5% CO2 and used in the test. The medium in each well was discarded, the cells were washed with an assay buffer (Ham's F-12 medium/0.5% BSA/20 mmol/l HEPES), and the assay buffer was added to each well. A compound to be tested having respective concentration was added thereto and incubated at room temperature for 40 minutes, and then the cells were washed 8 times with PBS (1 ml/well). The assay buffer was further added and incubated at 37°C for 4 hours. After 4 hours, the assay buffer was discarded and a radioisotope-labeled ligand was added. The culture mixture containing the cells was incubated at room temperature for 40 minutes and then washed three times with 1 ml/well of ice-cooled PBS. The cells were lysed with 1 mol/l sodium hydroxide aqueous solution, and radioactivity of the cell lysate was measured using a $\gamma$ counter.

As a result, SCH-351125 and TAK-779 showed IC50 values of 16.7 and 10.4 nmol/l, respectively. (3R)-1-Butyl-2,5-dioxo-3-((1R)-1-hydroxy-1-cyclohexylmethyl)-9-(4-(4-carboxyphenyloxy)phenylmethyl)-1,4,9-triazaspiro[5.5]undecane hydrochloride (compound 1) and (3R)-1-butyl-2,5-dioxo-3-((1R)-1-hydroxy-1-cyclohexylmethyl)-9-(4-(4-methylaminocarbonylphenyloxy)phenylmethyl)-1,4,9-triazaspiro[5.5]undecane hydrochloride (compound 2) showed IC50 values of 4.2 and 1.5 nmol/l, respectively. It was confirmed that compound 1 and compound 2 show strong inhibitory activities of about 4 times and about 2.5 times higher than those of SCH-351125 and TAK-779, respectively, in the case of compound 1, and about 11 times and about 7 times higher than the same in the case of compound 2.

Example 2

Anti-hCCR5 antibody binding inhibition test:

Human CCR5 expressing cells (hCCR5-CHO cells) were inoculated into a culture flask, cultured using a medium (Ham's F-12 medium/10% FBS/Antibiotic-Antimycotic) at 37°C for 2 to 3 days in a CO2 incubator of 5% CO2 and used in the test. The medium was removed from the flask, and the cells were peeled off and recovered by adding 1 mmol/l EDTA/PBS. The recovered cells were washed once with PBS and again suspended in PBS, and then the number of cells was counted. After centrifugation and subsequent removal of the supernatant by suction, the cells were suspended in an assay buffer (0.5% FBS/Ham's F-12 medium). Each of the compounds to be tested was added to the cell suspension and incubated on ice. An FITC-labeled anti-hCCR5 antibody (45531.111 or 45523.111) or an FITC-labeled isotype control antibody was further added thereto and allowed to undergo the reaction on ice under shading. After the reaction, the cells were washed twice with ice-cooled PBS and again suspended in ice-cooled FACS buffer (2% FBS/PBS), and the amount (fluorescence intensity) of the bound anti-hCCR5 antibody was measured using a flow cytometer (BECTON DICKINSON).

As a result, inhibitory effects of compound 1, compound 2, SCH-351125 and TAK-779 for the binding of anti-hCCR5 antibodies (45531.111 and 45523.111) were examined. The assay was carried out by adding 10 μmol/l of each compound and the results of flow cytometry were represented by a histogram, with the results shown in Fig. 1. Compound 1 and compound 2 showed strong action to inhibit binding of the anti-hCCR5 antibody 45531.111 to the hCCR5 expressing cells, but the inhibition by SCH-351125 and TAK-779 was weak. Also, compound 1, compound 2 and SCH-351125 strongly inhibited binding of 45523.111, but TAK-779 showed weak inhibition. Based on the above results, it was found that the inhibitory activity upon the hCCR5 binding of both antibodies was strong in order of compound 1 = compound 2 > SCH-351125 > TAK-779.

Example 3

Inhibitory action of SCH-351125 and TAK-779 upon the hCCR5 binding of tritium-labeled compound 1:

Human CCR5 expressing cells (hCCR5-CHO cells) were inoculated into a culture plate, cultured using a medium (Ham's F-12 medium/10% FBS/Antibiotic-Antimycotic) at 37°C for 1 day in a CO2 incubator of 5% CO2 and used in the test. The medium in each well was discarded, the cells were washed with an assay buffer (Ham's F-12 medium/0.5% BSA/20 mmol/l HEPES), and the assay buffer was added thereto. Respective amount of SCH-351125 or TAK-779 was added, and 3 doses of tritium-labeled compound 1 solution was added thereto. After incubation at room temperature for 2 hours, the cells were washed with 1 ml/well of ice-cooled D-PBS. The cells were lysed with 1 mol/l NaOH and neutralized, and then a scintillator (ACS II, Amersham) was added thereto. Radioactivity of each vial was measured using a liquid scintillation counter (TRI-CARB 2900TR, Perkin Elmer Life Sciences).

Concentration-displacement curves of SCH-351125 and TAK-779 at respective concentrations of the tritium-labeled compound 1 were prepared to examine the action mode. As a result, each of SCH-351125 and TAK-779 showed complete inhibition of the hCCR5 binding of tritium-labeled compound 1 when the concentration was increased, and the displacement curves shifted in parallel to the high concentration side as the concentration of tritium-labeled compound 1 increased. Based on this result, it was confirmed that compound 1 competitively antagonizes with SCH-351125

and TAK-779, and that the compound 1 recognizes not a site completely different from SCH-351125 and TAK-779 but a partially overlapped region.

Example 4

Inhibitory action upon cell migration:

A migration test induced by MIP-1α was carried out using human CCR5 expressing cells (hCCR5-Ba/F3 cells). Firstly, 0.5 ml of a medium containing MIP-1α was added to the lower chamber (transwell bottom) of a 24 well transwell plate. Next, 2 times concentration of compound 1, SCH-351125 or TAK-779 as the compound to be tested was added in 50 μl portions to the upper lower chamber (transwell insert) of the 24 well transwell plate. Subsequently, 50 μl of an hCCR5-Ba/F3 cell suspension was added to the transwell insert. The migration was started by moving the upper chamber wells of the transwell plate to the lower chamber wells containing chemokine. These cells were cultured in a CO2 incubator (37°C, 5% CO2, 95% humidity) for 3 hours, and then lower part of the transwell insert was washed out into the lower chamber using 0.5 ml of a washing buffer (PBS, 0.1% FBS, 2 mmol/l EDTA). Measurement of the number of cells was carried out using a flow cytometer (BECTON DICKINSON), and the number of cells inside the gate (FSC × SSC) was counted. The number of migrated cells was defined as their ratio (%) to the count when the firstly added cells were analyzed by FACS, and the IC50 value was calculated from the inhibitory activity for the DMSO control group.

As a result, compound 1, SCH-351125 and TAK-779 inhibited migration of the hCCR5-Ba/F3 cells by MIP-1α, and their IC50 values were 4.3, 21.0 and 55.0 nmol/l, respectively.

Compound 1, SCH-351125 and TAK-779 inhibited the cell migration function by inhibiting binding of MIP-1α to hCCR5. The degree of inhibition of the functional assay was compound 1 > SCH-351125 > TAK-779 in that order.

Example 5

Persistency of hCCR5 binding in the living body of mouse:

Human CCR5 expressing cells (hCCR5-Ba/F3 cells) were suspended in 0.1% BSA/PBS, carboxyfluorescein diacetate succinimidyl ester (CFSE, final concentration 1 μmol/l) was added thereto, and the mixture was incubated at 37°C under shade. The cells were washed twice with 0.1% BSA/PBS and suspended in 0.5% BSA/Ham's F-12 medium. Sufficient amount of compound 1 was added thereto and incubated at 37°C under shade, thereby effecting binding of compound 1 to hCCR5. The vehicle group was treated by the same operation using 0.1% DMSO alone. In order to remove free compound 1, the cells were washed twice with PBS and suspended in PBS. This cell suspension was transferred into tail vein of Balb/c mouse, and a blood sample was collected from abdominal vena cava and the spleen was removed under ether anesthesia after several hours(e.g., 0, 0.5, 1.5, 3 or 5 hours) of the cell transfer. The peripheral blood was centrifuged using Lymphocyte-M to prepare PBMC, and the spleen was crushed, subjected to hemolytic treatment and then washed to obtain cells. The thus prepared cells were stained with PE-labeled anti-hCCR5 antibodies (2D7 and 45531.111). CFSE-positive hCCR5-Ba/F3 cells were detected by a flow cytometer, and fluorescence intensity (mean value) of the PE-labeled anti-hCCR5 antibody bound to the cells was measured to calculate the occupying ratio by the following formulae.

Δ mean value = (fluorescence intensity mean value at the time of 45531.111 binding of a sample to be examined) - (fluorescence intensity mean value of cells recovered from a mouse for isotype control and stained with PE-labeled isotype control)

Correction value = (fluorescence intensity Δ mean value at the time of 45531.111 binding of the sample to be examined) / (fluorescence intensity Δ mean value at the time of 2D7 binding of the sample to be examined)

Receptor occupying ratio (R) = {1-((correction value of the sample to be examined) / (mean value of the

correction value of vehicle))} × 100

Corrected receptor occupying ratio = {receptor occupying ratio (R) / (mean value of the receptor occupying ratio (R)of vehicle at 0 hour)} × 100

Persistency of hCCR5 binding of compound 1 in living mouse was examined. The hCCR5-occupying ratio (%) of compound 1 at 0, 0.5, 1.5, 3 or 5 hours after the cell transfer is shown in Fig. 2. It was confirmed that compound 1 binds to hCCR5 for a prolonged period of time in the living body of mouse and shows 61% of hCCR5-occupying ratio even after 5 hours of the cell transfer. Its half-life was 12 hours according to this periodical change, and it was confirmed that compound 1 remains on hCCR5 for a prolonged period of time even when the compound is not present in the circulating blood.

Example 6

Persistency of hCCR5 binding in the living body of mouse after compound administration:

Compound 1 was administered into abdominal cavity at a dose of 3 or 30 mg/kg, and 15 minutes thereafter, CFSE-labeled hCCR5-Ba/F3 cells were transferred into tail vein of Balb/c mouse, and a blood sample was collected from abdominal vena cava and the spleen was removed under ether anesthesia after several hours(0, 0.5, 4 or 8 hours) of the cell transfer. The peripheral blood was centrifuged using Lymphocyte-M to prepare PBMC, and the spleen was crushed, subjected to hemolytic treatment and then washed to obtain cells. The thus prepared cells were stained with PE-labeled anti-hCCR5 antibody (45531.111). CFSE-positive hCCR5-Ba/F3 cells were detected by a flow cytometer, and fluorescence intensity (mean value) of PE-labeled anti-hCCR5 antibodies (2D7 and 45531.111) bound to the cells was measured.

The occupying ratio was calculated by the following formulae.

Δ mean value = (fluorescence intensity mean value at the time of 45531.111 binding of a sample to be examined)-(fluorescence intensity mean value of cells recovered from a mouse for isotype control and stained with PE-labeledisotype control)

Correction value = (fluorescence intensity Δ mean value at the time of 45531.111 binding of the sample to be examined)/ (fluorescence intensity Δ mean value at the time of 2D7 binding of the sample to be examined)

Receptor occupying ratio (R) = {1-((correction value of the sample to be examined) / (mean value of the correction value of vehicle))} ×100

As a result, it was confirmed that the hCCR5-occupying ratio of compound 1 in the peripheral blood after 0.5, 4 or 8 hours of the cell transfer was 96%, 85% or 70% at a dose of 30 mg/kg, and 93%, 68% or 54% at a dose of 3 mg/kg, respectively. Also, in the case of the spleen, it was revealed that 96%, 69% and 54% of hCCR5 were occupied by compound 1 after 0.5, 4 and 8 hours at a dose of 30 mg/kg, respectively, and 92%, 55% and 32% after 0.5, 4 and 8 hours at a dose of 3 mg/kg, respectively. The blood plasma levels of compound 1 in this test were 1612, 22 and 4 nmol/l after 0.5, 4 and 8 hours at a dose of 30 mg/kg, respectively, and 352, 5 and 1 nmol/l after 0.5, 4 and 8 hours at a dose of 3 mg/kg, respectively. When hCCR5-occupying ratios deduced from these plasma levels were calculated based on an in vitro mouse plasma antibody inhibition test, it was revealed that they are about 100%, 52% and 13% after 0.5, 4 and 8 hours at a dose of 30 mg/kg, respectively, and 100%, 16% and 3% after 0.5, 4 and 8 hours at a dose of 3 mg/kg, respectively. Based on this, it was revealed that compound 1 binds to hCCR5 in the living body, and the

binding is maintained at a level higher than the occupying ratio deduced from its blood plasma concentration.

Based on the above results, it was confirmed that the compound 1 shows binding persistency in the living body. Since such a compound which can continuously bind to hCCR5 for a prolonged period of time even in the circulating blood, namely a compound which binds to a strong binding site, can continue its action, it is evident that its pharmacological effects can be continued by administering it orally or parenterally, so that there is a merit of being able to reduce administration frequency of pharmaceutical preparations.

Example 7

Action to inhibit HIV-1 infection upon human PBMC:

Human PBMC (peripheral blood mononuclear cell) was isolated from an HIV-negative healthy person by Ficol-Hipaque density gradient centrifugation and cultured for 3 days in the presence of 10 μg/ml of PHA (phytohemagglutinin). The PHA-stimulated PBMC was suspended in RPMI 1640 containing 10% serum and inoculated into a 96 well microplate. This was further exposed to an R5-HIV-1 strain (e.g., HTV-1BaL, *etc.*) in the presence of a CCR5 antagonist alone having varied concentration. After 7 days of culturing, amount of HIV-1p24 antigen in the culture supernatant was measured by an EIA method using Lumipulse F (Fujirebio).

Example 8

Method for judging CCR5 agonist or antagonist;

(8-1): Establishment of human CCR5 expression cell

<8-1-A> Isolation of human CCR5 gene

Human placenta cDNA was prepared using Marathon cDNA amplification kit (Clontech). PCR primers hCCR5XbaI-F1: 5'-AGCTAGTCTA GATCCGTTCC CCTACAAGAA ACTCTCC-3' (SEQ ID NO:1) and hCCR5XbaI-R1: 5'-AGCTAGTCTA GAGTGCACAA CTCTGACTGG GTCACCA-3' (SEQ ID NO:2) were designed based on the sequence of GenBank U54994.

Using the human placenta cDNA as the template, PCR (2 minutes at 95°C → [30 seconds at 95°C, 45 seconds at 60°C, 1 minute at 72°C] × 35 times) was carried out using Ex Taq (Takara). The amplified PCR product was subjected to 1% agarose gel electrophoresis, purified using QIAquick Gel Extraction Kit (QIAGEN) and then digested with *Xba*I. The digested fragment was connected to an expression vector pEF-BOS-bsr using DNA Ligation Kit Ver. 2 (Takara) and transformed into Escherichia coli DH5α. By using the resulting plasmid pEF-BOS-bsr/hCCR5, the DNA sequence was verified.

<8-1-B> Culturing of CHO cell

CHO-dhfr(-) was cultured in a carbon dioxide incubator (temperature: 37°C, CO2 concentration: 5%, humidity: 95%) using Ham's F-12 (containing fetal bovine serum (10%), penicillin (50 U/ml) and streptomycin (50 mg/ml)). Also, the transduced cells were cultured by adding blasticidin (5 μg/ml) to the above medium.

<8-1-C> Transduction into CHO cell

The plasmid pEF-BOS-bsr/hCCR5 transduced into CHO-dhfr(-) using DMRIE-C reagent (Gibco BRL). After 48 hours thereof, selection was carried out by changing the medium to a medium containing 5 μg/ml of blasticidin, thereby establishing a stable over-expression cell.

<8-1-D> Analysis of CCR5 expression

Human CCR5 expression strength in the clone obtained by the method described in the above <8-1-C> was analyzed by detecting the cell by a fluorescein isothiocyanate (FITC)-labeled anti-CCR5 antibody (BD Pharmingen) and measuring using FACSort (registered trademark, manufactured by Becton Dickinson). In this case, FITC-labeled mouse IgG 2aκ (BD Pharmingen) was used as the isotype control antibody.

(8-2): Monitoring of increase of intracellular Ca concentration via CCR5

<8-2-A> Effect of CCR5 agonist to increase intracellular Ca concentration and effect of CCR5 antagonist to inhibit increase of intracellular Ca concentration by endogenous ligand

The human CCR5 stable expression CHO cell prepared in the above (8-1) was inoculated in $3.5\times10^4$ cells/100 μl/well portions. On the next day, the medium was changed to 80 μl/well of an assay buffer (Ham's F-12 containing Fura-2AM (5 μM), probenecid (2.5 mM) and HEPES (20 mM, pH 7.4)) and cultured at 37°C for 1 hour, and then the cells were washed twice with a washing buffer (containing Hanks (9.8 mg/ml), HEPES (20 mM) and probenecid (2.5 mM)), mixed with 100 μl of the washing buffer and allowed to stand at room temperature for 30 minutes. Compounds (0.01, 0.03, 0.1, 0.3, 1, 3, 10 or 30 μM for each) were added thereto, and MIP-1α (30 nM in final concentration) was added thereto 3 minutes thereafter. Monitoring was started 30 seconds before the compound addition, and increase of intracellular Ca concentration was monitored for 7.5 minutes. Regarding the intracellular Ca concentration, a signal strength of excitation light 340 nm/380 nm was measured using Fluorescence Drug Screening System 4000 (manufactured by Hamamatsu Photonics). Agonist and antagonist activities of test compounds were calculated by the following formulae using the signal strength of MIP-1α at the time of no addition of compound as 100%.

Agonist activity (%) = (Ea/Ec) ×100

Ec: Measured value of the transient Ca2+ increase at the time of the addition of MIP-1α
Ea: Measured value of the transient Ca2+ increase at the time of the addition of test compound

Antagonist activity (%) = {(Ec-Ea)/Ec} × 100

Ec: Measured value of the transient Ca2+ increase at the time of the addition of MIP-1α after the addition of vehicle containing no test compound
Ea: Measured value of the transient Ca2+ increase by MIP-1α after the addition of test compound

<8-2-B> Action inhibition of CCR5 agonist by endogenous ligand

In the same manner as the above <8-2-A>, action inhibition activity of CCR5 agonist by endogenous ligand was examined. A CCR5 endogenous ligand MIP-1α (30 nM) was added, each agonist (10 μM) was added 3 minutes thereafter, and intracellular Ca concentration (signal strength) was monitored for 7.5 minutes from 30 seconds before the start of MIP-1α addition.

Each CCR5 agonist by itself increased intracellular Ca concentration (signal strength) of CCR5, but when the CCR5 endogenous ligand MIP-1α was added in advance, desensitization of CCR5 occurred and increase of the intracellular Ca concentration (signal strength) by the compound of the present invention was inhibited. Accordingly, it was shown that these compounds specifically activate CCR5.

Example 9

Migration test of human CCR5 expression cell (hCCR5-Ba/F3 cell):

(9-1): Establishment of human CCR5 expression cell

<9-1-A> Isolation of human CCR5 gene

This was carried out by the method described in the above <8-1-A> Isolation of human CCR5 gene.

<9-1-B> Culturing of Ba/F3 cells

Ba/F3 cells were statically cultured in a carbon dioxide incubator (temperature: 37°C, CO2 concentration, 5%, humidity: 95%) using RPMI-1640 medium (Gibco BRL) containing an antibiotic preparation (Antibiotic-Antimycotic) (final concentration: penicillin G sodium (100 U/ml), streptomycin sulfate (100 μg/ml) and amphotericin B (0.25 μg/ml))

(Gibco BRL), fetal bovine serum (FBS) (10%) and interleukin 3 (IL-3) (5 ng/ml) (Pepro Tech, Inc.). For the culturing of foreign gene stably over-expressing cells, blasticidin (Kaken Pharmaceutical) was added to the above medium to a final concentration of 10 μg/ml.

<9-1-C> Transduction into Ba/F3 cells

A plasmid for human CCR5 expression (pEF-BOS-bsr/hCCR5) was made into straight chain by digesting with *Aat*II. The straightened plasmid was purified using QIA Quick PCR Purification Kit (QIAGEN) and then transduced into Ba/F3 cells by electroporation (Gene Pulser (BIO RAD) 960 μF/250 V). The cells were inoculated into a 96 well culture plate at a density of 1,000, 100 or 10 cells/well, and 48 hours thereafter, blasticidin was added thereto to a final concentration of 10 μg/ml to clone blasticidin-resistant strains, thereby establishing a stable over-expression clone (hCCR5-Ba/F3 cell) which expresses the transduced foreign gene.

<9-1-D> Analysis of CCR5 expression

This was carried out by the method described in the above <8-1-D>.

(9-2): Cell migration test

Migration ability of Ba/F3 cells expressing human CCR5 for CCR5 agonist was examined. Firstly, 0.01, 0.03, 0.1, 0.3 or 1 μM of a test compound (0.5 ml/well) was added to the lower chamber of a 24 well transwell plate, and hCCR5-Ba/F3 cells ($1\times10^5$ cells/0.05 ml/well) was added to the upper chamber. The test was started by superposing the upper chamber on the lower chamber, and the cells were incubated for 3 hours in a carbon dioxide incubator (temperature: 37°C, CO2 concentration: 5%, humidity: 95%). In addition, in order to carry out relative evaluation of the number of these migration cells, a standard sample in the case of the migration of all of the initially added cells (a chemokine-containing medium of $1\times10^5$ cells/0.5 ml/well) was prepared, and this was also incubated in the same manner. Cells on the outside bottom part of the upper chamber were washed out into the lower chamber using 0.5 ml of a washing buffer (phosphate buffered saline (PBS) containing sodium ethylenediaminetetraacetate (EDTA) (2 mM) and FBS (0.1%)), and the cells migrated into the lower chamber were recovered in an FACS tube. A 0.5 ml portion of the washing buffer was also added regarding the standard. The number of cells in the standard sample and that in the lower chamber were measured using FACSort (registered trademark, manufactured by Becton Dickinson), and the number of cells within a 30 seconds of gate (FSC × SSC) was counted.

Migration activity by the test compound at each concentration was calculated in accordance with the following formula, and the result was shown as a mean value of n = 2.

Migration activity (%) = (B/A) × 100

A: Measured value of standard sample
B: Measured value of test compound-added sample

SEQ
## Claims

1. An antagonist or an agonist which binds to a strong binding site of CCR5, excluding the compounds described in WO01/40227, WO02/74769 and WO02/74770 and SCH-351125.

2. A preventive and/or therapeutic agent for an allergic disease, an inflammatory disease, an immune disease and/or a cancer, which comprises the antagonist or the agonist according to claim 1.

3. The preventive and/or therapeutic agent according to claim 2, wherein the allergic disease, the inflammatory disease, the immune disease and the cancer are diseases selected from asthma, atopic dermatitis, nettle rash, allergic bronchopulmonary aspergillosis, allergic eosinophilic gastroenteritis, nephritis, nephropathy, hepatitis, arthritis, rheumatoid arthritis, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplanted organ rejection reaction, immunosuppression, cancer metastasis, HIV infection and acquired immunodeficiency syndrome.

4. The preventive and/or therapeutic agent according to claim 2, wherein the immune disease is HIV infection, acquired immunodeficiency syndrome and/or transplanted organ rejection reaction.

5. A method for screening a compound which binds to a strong binding site of CCR5, which comprises

(a) allowing a CCR5 expressing cell or a membrane fraction thereof to contact with a compound to be tested,

(b) washing the cell or the membrane from 1 to 12 times, and
(c) adding a labeled ligand and measuring amount of the bound labeled ligand.

**6.** The method according to claim 5, wherein the cell or the membrane is washed from 6 to 10 times.

**7.** A method for screening a compound which binds to a strong binding site of CCR5, which comprises

(a) allowing a CCR5 expressing cell or a membrane fraction thereof to contact with a compound to be tested,
(b) allowing the test compound-bound cell or a membrane fraction thereof prepared in the above (a) to contact with a labeled anti-CCR5 antibody, and
(c) measuring the labeled anti-CCR5 antibody bound to the CCR5 expressing cell or a membrane fraction thereof.

**8.** The method according to claim 7, wherein the anti-CCR5 antibody is 45531.111 antibody and/or 45523.111 antibody.

**9.** A method for measuring an occupying ratio of a compound bound to a CCR5 expressing cell or a membrane fraction thereof, which comprises

(a) allowing a CCR5 expressing cell or a membrane fraction thereof to contact with a compound to be tested,
(b) allowing the test compound-bound cell or a membrane fraction thereof prepared in the above step (a) to contact with a labeled anti-CCR5 antibody, and
(c) calculating an occupying ratio of the compound bound to CCR5 on the cell or membrane fraction thereof, based on the ratio of a bound amount of the anti-CCR5 antibody when the compound is bound to CCR5 to a bound amount of the anti-CCR5 antibody when the compound is not bound to CCR5, which is defined as 100%.

**10.** A method for periodically monitoring an occupying ratio of a compound which binds to a binding site on a CCR5 expressing cell in blood, which comprises

(a) administering a compound which binds to a binding site of CCR5 to a mammal and then separating a cell population containing a cell expressing CCR5 in blood,
(b) allowing the separated cell population to contact with a labeled anti-CCR5 antibody, and
(c) calculating an occupying ratio of the compound which binds to a binding site of CCR5 on the separated cell, based on the ratio of a bound amount of the anti-CCR5 antibody when the compound is bound to CCR5 to a bound amount of the anti-CCR5 antibody when the compound is not bound to CCR5, which is defined as 100%.

**11.** The method according to claim 10, wherein the anti-CCR5 antibody is 45531.111 antibody and/or 45523.111 antibody.

**12.** A method for determining a dose and an administration frequency which show such an efficacy that an inhibition ratio of about 50% or 90% can be obtained in administering a compound which binds to a binding site of CCR5, which comprises:

(a) measuring IC50 value or IC90 value of the inhibitory activity of a compound which binds to a binding site of CCR5, by an in vitro activity measuring method,
(b) calculating an occupying ratio of a compound which binds to a binding site of CCR5 on a CCR5 expressing cell at a compound concentration corresponding to the IC50 value or IC90 value described in the above (a), by the method according to claim 9, and
(c) comparing the occupying ratio of a compound which binds to a binding site of CCR5, obtained at each dose and monitoring time by the monitoring method according to claim 10, with the occupying ratio obtained by the method described in the above (b).

**13.** The method according to claim 12, wherein the in vitro activity measuring method is an anti-HIV activity measuring method.

**14.** A preventive and/or therapeutic agent for an allergic disease, an inflammatory disease, an immune disease and/or a cancer, which comprises a compound which binds to a strong binding site of CCR5 selected by the method

according to any one of claim 5, 7, 9, 10 and 12 as an active ingredient.

**15.** The preventive and/or therapeutic agent according to claim 14, wherein the immune disease is HIV infection, acquired immunodeficiency syndrome and/or transplanted organ rejection reaction.

**16.** The preventive and/or therapeutic agent according to claim 2 or 14,
wherein the administration method is to administer it orally or parenterally at intervals of one day, two days, three days or several days.

**17.** The preventive and/or therapeutic agent according to claim 16, wherein the immune disease is HIV infection, acquired immunodeficiency syndrome and/or transplanted organ rejection reaction.

**18.** A method for screening an antagonist or an agonist which binds to a strong binding site of CCR5, which comprises administering it orally or pareziterally at intervals of one day, two days, three days or several days.

**19.** An antagonist or an agonist which binds to a strong binding site of a chemokine receptor.

**20.** A preventive and/or therapeutic agent for an allergic disease, an inflammatory disease, an immune disease and/or a cancer, which comprises the antagonist or the agonist according to claim 19.

**21.** The preventive and/or therapeutic agent according to claim 20, wherein the allergic disease, inflammatory disease, immune disease and cancer are diseases selected from asthma, atopic dermatitis, nettle rash, allergic bronchopulmonary aspergillosis, allergic eosinophilic gastroenteritis, nephritis, nephropathy, hepatitis, arthritis, rheumatoid arthritis, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplanted organ rejection reaction, immunosuppression, cancer metastasis, HIV infection and acquired immunodeficiency syndrome.

**22.** A method for screening a compound which binds to a strong binding site of a chemokine receptor, which comprises

(a) allowing a chemokine receptor expressing cell or a membrane fraction thereof to contact with a compound to be tested,
(b) washing the cell or the membrane from 1 to 12 times, and
(c) adding a labeled ligand and measuring an amount of the bound labeled ligand.

**23.** A method for screening a compound which binds to a strong binding site of a chemokine receptor, which comprises

(a) allowing a chemokine receptor expressing cell or a membrane fraction thereof to contact with a compound to be tested,
(b) allowing the test compound-bound cell or a membrane fraction thereof prepared in the above step (a) to contact with a labeled anti-chemokine receptor antibody, and
(c) measuring the labeled anti-chemokine receptor antibody bound to the chemokine receptor expressing cell or a membrane fraction thereof

**24.** A preventive and/or therapeutic agent for an allergic disease, an inflammatory disease, an immune disease and/or a cancer, which comprises a compound which binds to a strong binding site of a chemokine receptor selected by the method according to claim 22 or 23 as an active ingredient.

**25.** A method for preventing and/or treating a CCR5 intervening disease in a mammal, which comprises administering an effective amount of the agonist or the antagonist according to claim 1.

**26.** A method for preventing and/or treating a chemokine receptor intervening disease in a mammal, which comprises administering an effective amount of the antagonist or the agonist according to claim 19.

**27.** A method for preventing and/or treating an allergic disease, an inflammatory disease, an immune disease and/or a cancer, which comprises administering a compound which binds to a strong binding site of CCR5 selected by the method according to any one of claims 5, 7, 9, 10 and 12 as an active ingredient.

**28.** Use of the agonist and/or the antagonist according to claim 1 for producing a preventive and/or therapeutic agent for a CCR5 intervening disease.

**29.** Use of the agonist and/or the antagonist according to claim 19 for producing a preventive and/or therapeutic agent for a chemokine receptor intervening disease.

**30.** Use of a compound which binds to a strong binding site of CCR5, which is selected by the method according to any one of claims 5, 7, 9, 10 and 12, for producing a preventive and/or therapeutic agent for a CCR5 intervening disease.

FIG. 1

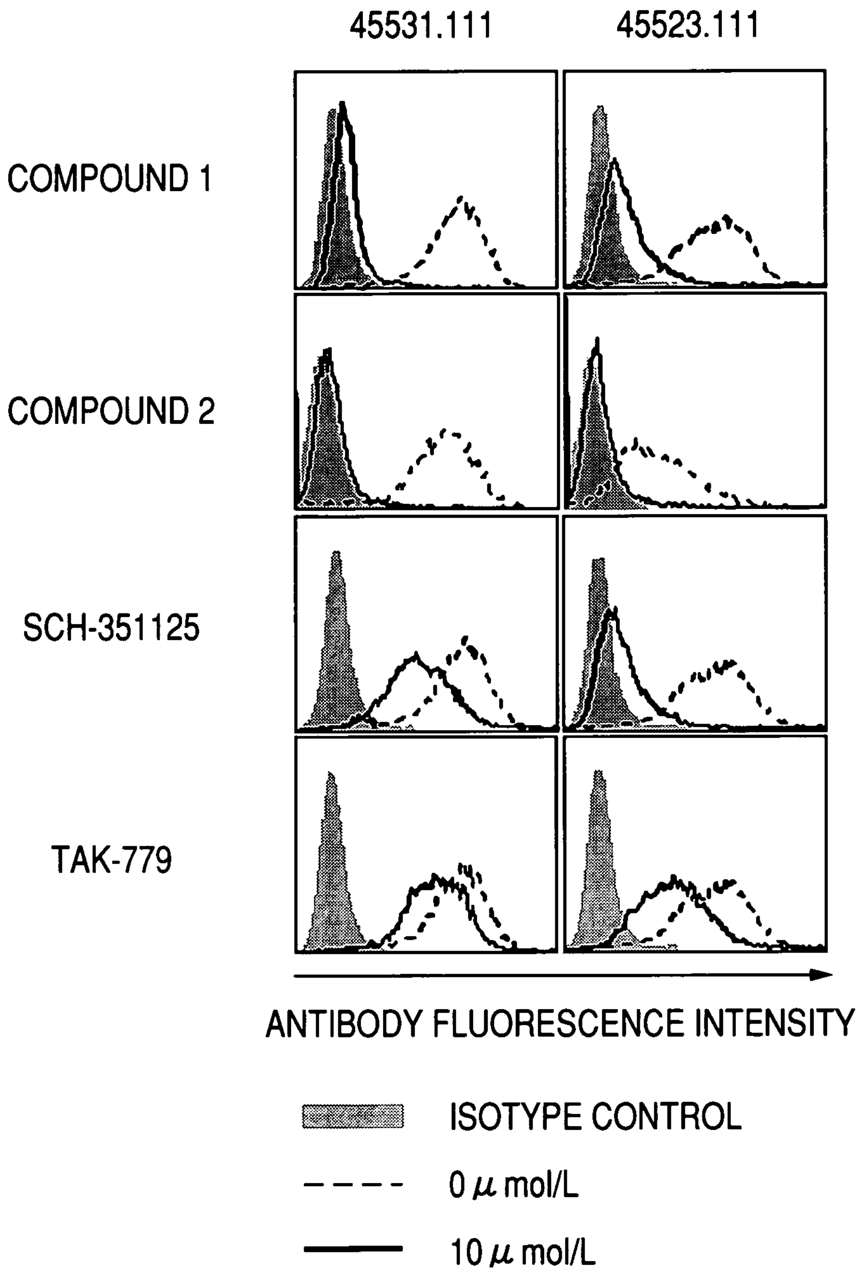
45531.111
45523.111
COMPOUND 1
COMPOUND 2
SCH-351125
TAK-779
ANTIBODY FLUORESCENCE INTENSITY
ISOTYPE CONTROL
0 μ mol/L
10 μ mol/L

FIG. 2

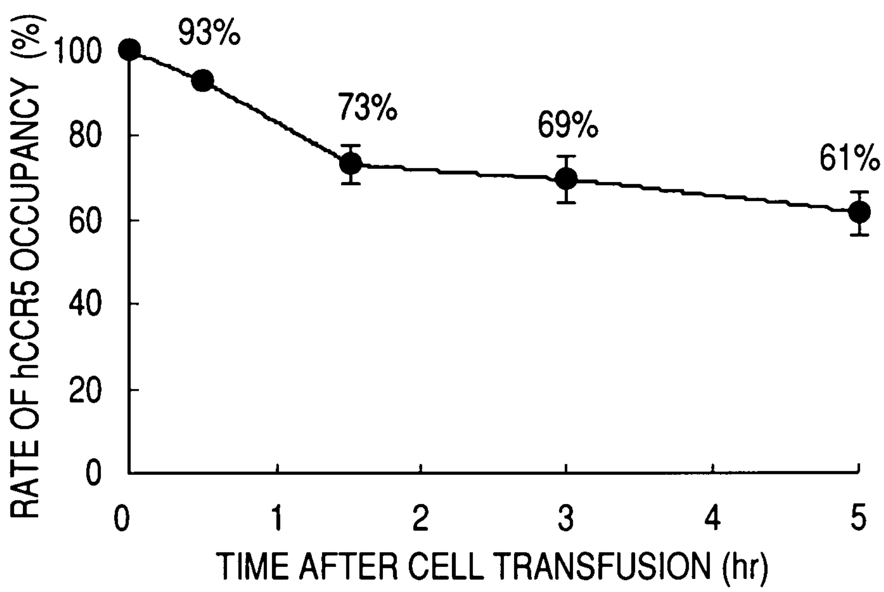
RATE OF hCCR5 OCCUPANCY (%)
100
80
60
40
20
0
93%
73%
69%
61%
0
1
2
3
4
5
TIME AFTER CELL TRANSFUSION (hr)

## INTERNATIONAL SEARCH REPORT

International application No.
PCT/JP03/15973

A. CLASSIFICATION OF SUBJECT MATTER
Int.Cl[7] A61K45/00, 31/49, 31/503, 31/5377, A61P1/04, 3/10, 9/00, 11/00, 13/12, 17/00, 25/00, 27/02, 29/00, 31/04, 31/18, 35/00, 37/02, 43/00

According to International Patent Classification (IPC) or to both national classification and IPC

B. FIELDS SEARCHED

Minimum documentation searched (classification system followed by classification symbols)
Int.Cl[7] A61K45/00-08, 31/00-33/44

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)
CAPLUS(STN), BIOSIS(STN), REGISTRY(STN), EMBASE(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| X | WO 02/074769 A1 (ONO PHARMACEUTICAL, CO., LTD.), 26 September, 2002 (26.09.02), Full text; in particular, examples 75(50), 75(54) & EP 1378509 A1 | 14-17,19-21, 24,29,30 |
| X | WO 02/074770 A1 (ONO PHARMACEUTICAL, CO., LTD.), 26 September, 2002 (26.09.02), Full text; in particular, examples 9(50), 9(54) & EP 1378510 A1 | 14-17,19-21, 24,29,30 |
| X | EP 1236726 A1 (ONO PHARMACEUTICAL, CO., LTD.), 04 September, 2002 (04.09.02), Full text & WO 01/40227 A1 | 14-17,19-21, 24,29,30 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>05 February, 2004 (05.02.04) | Date of mailing of the international search report<br>17 February, 2004 (17.02.04) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/15973

C (Continuation). DOCUMENTS CONSIDERED TO BE RELEVANT

| Category* | Citation of document, with indication, where appropriate, of the relevant passages | Relevant to claim No. |
|---|---|---|
| X | WO 00/10965 A2 (TAKEDA CHEMICAL INDUSTRIES, LTD.), 02 March, 2000 (02.03.00), Full text & JP 2000-128782 A | 1-9,12-24, 28-30 |
| X | WO 00/37455 A1 (TAKEDA CHEMICAL INDUSTRIES, LTD.), 29 June, 2000 (29.06.00), Full text & JP 2001-58988 A | 1-9,12-24, 28-30 |
| X | WO 00/66558 A1 (SCHERING CORP.), 09 November, 2000 (09.11.00), Full text & JP 2002-543185 A | 1-9,12-24, 28-30 |
| Y | WO 98/18826 A2 (LEUKOSITE ICN.), 07 May, 1998 (07.05.98), Full text (Family: none) | 7-9,18,23,30 |

Form PCT/ISA/210 (continuation of second sheet) (July 1998)

## INTERNATIONAL SEARCH REPORT

International application No.
PCT/JP03/15973

**Box I Observations where certain claims were found unsearchable (Continuation of item 2 of first sheet)**

This international search report has not been established in respect of certain claims under Article 17(2)(a) for the following reasons:

1. [X] Claims Nos.: 10, 11, 25 to 27
   because they relate to subject matter not required to be searched by this Authority, namely:
   Claims 10, 11, 25 to 27 pertain to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)